# EUROPEAN PATENT SPECIFICATION

(11) **EP 4 058 452 B1**
(45) Date of publication and mention of the grant of the patent: **09.07.2025**
(21) Application number: 20804515.3
(22) Date of filing: 10.11.2020
(51) Int. Cl.: C07D 471/04, A61P 37/00, C07D 519/00, A61K 31/4985

(54) **HYDROPYRAZINO[1,2-B]ISOQUINOLINE COMPOUNDS FOR THE TREATMENT OF AUTOIMMUNE DISEASE**
HYDROPYRAZINO[1,2-B]ISOCHINOLINVERBINDUNGEN ZUR BEHANDLUNG VON AUTOIMMUNERKRANKUNGEN
COMPOSÉS D'HYDROPYRAZINO[1,2-B]ISOQUINOLINE POUR LE TRAITEMENT D'UNE MALADIE AUTO-IMMUNE

(30) Priority: 12.11.2019 WO PCT/CN2019/117514
(43) Date of publication of application: 21.09.2022
(73) Proprietor: F. Hoffmann-La Roche AG, 4070 Basel (CH)
(72) Inventor: DEY, Fabian, 4070 Basel (CH); DING, Dong, Shanghai, 201203 (CN); XU, Hongtao, Shanghai, 201203 (CN); ZHU, Wei, Shanghai, 201203 (CN); ZOU, Ge, Shanghai, 201203 (CN)
(74) Representative: Belkacemi, Doria
(86) International application number: PCT/EP2020/081614
(87) International publication number: WO 2021/094301

(56) References cited:
- WO-A1-2017/106607
- WO-A1-2019/233941
- WO-A1-2020/207991

## Description

The present invention relates to organic compounds useful for therapy and/or prophylaxis in a mammal, and in particular to antagonist of TLR7 and/or TLR8 and/or TLR9 useful for treating systemic lupus erythematosus or lupus nephritis.

### FIELD OF THE INVENTION

Autoimmune connective tissue disease (CTD) include prototypical autoimmune syndromes such as Systemic Lupus Erythematosus (SLE), primary Sjögren's syndrome (pSjS), mixed connective tissue disease (MCTD), Dermatomyositis/Polymyositis (DM/PM), Rheumatoid Arthritis (RA), and systemic sclerosis (SSc). With the exception of RA, no really effective and safe therapies are available to patients. SLE represents the prototypical CTD with a prevalence of 20-150 per 100,000 and causes broad inflammation and tissue damage in distinct organs, from commonly observed symptoms in the skin and joints to renal, lung, or heart failure. Traditionally, SLE has been treated with nonspecific anti-inflammatory or immunosuppressive drugs. However, long term usage of immunosuppressive drug, e.g. corticosteroids is only partially effective, and is associated with undesirable toxicity and side effects. Belimumab is the only FDA-approved drug for lupus in the last 50 years, despite its modest and delayed efficacy in only a fraction of SLE patients (Navarra, S. V. et al Lancet 2011, 377, 721.). Other biologics, such as anti-CD20 mAbs, mAbs against or soluble receptors of specific cytokines, have failed in most clinical studies. Thus, novel therapies are required that provide sustained improvement in a greater proportion of patient groups and are safer for chronic use in many autoimmune as well as autoinflammation diseases.

Toll like Receptors (TLR) are an important family of pattern recognition receptors (PRR) which can initiate broad immune responses in a wide variety of immune cells. As natural host defense sensors, endosomal TLRs 7, 8 and 9 recognize nucleic acids derived from viruses, bacteria; specifically, TLR7/8 and TLR9 recognize single-stranded RNA (ssRNA) and single-stranded CpG-DNA, respectively. However, aberrant nucleic acid sensing of TRL7,8,9 is considered as a key node in a broad of autoimmune and auto-inflammatory diseases (Krieg, A. M. et al. Immunol. Rev. 2007, 220, 251. Jiménez-Dalmaroni, M. J. et al Autoimmun Rev. 2016, 15, 1. Chen, J. Q., et al. Clinical Reviews in Allergy & Immunology 2016, 50, 1.). Anti-RNA and anti-DNA antibodies are well established diagnostic markers of SLE, and these antibodies can deliver both self-RNA and self-DNA to endosomes. While self-RNA complexes can be recognized by TLR7 and TLR8, self-DNA complexes can trigger TLR9 activation. Indeed, defective clearance of self-RNA and self-DNA from blood and/or tissues is evident in SLE (Systemic Lupus Erythematosus) patients. TLR7 and TLR9 have been reported to be upregulated in SLE tissues, and correlate with chronicity and activity of lupus nephritis, respectively. In B cells of SLE patients, TLR7 expression correlates with anti-RNP antibody production, while TLR9 expression with IL-6 and anti-dsDNA antibody levels. Consistently, in lupus mouse models, TLR7 is required for anti-RNA antibodies, and TLR9 is required for anti-nucleosome antibody. On the other hand, overexpression of TLR7 or human TLR8 in mice promotes autoimmunity and autoinflammation. Moreover, activation of TLR8 specifically contributes to inflammatory cytokine secretion of mDC/macrophages, neutrophil NETosis, induction of Th17 cells, and suppression of Treg cells. In addition to the described role of TLR9 in promoting autoantibody production of B cells, activation of TLR9 by self-DNA in pDC also leads to induction of type I IFNs and other inflammatory cytokines. Given these roles of TLR9 in both pDC and B cells, both as key contributors to the pathogenesis of autoimmune diseases, and the extensive presence of self-DNA complexes that could readily activate TLR9 in many patients with autoimmune diseases, it may have extra benefit to further block self-DNA mediated TLR9 pathways on top of inhibition of TLR7 and TLR8 pathways. Taken together, TLR7, 8, and 9 pathways represent new therapeutic targets for the treatment of autoimmune and auto-inflammatory diseases, for which no effective steroid-free and non-cytotoxic oral drugs exist, and inhibition of all these pathways from the very upstream may deliver satisfying therapeutic effects. As such, we invented oral compounds that target and suppress TLR7, TLR8 and TLR9 for the treatment of autoimmune and auto-inflammatory diseases WO2017106607 discloses polycyclic TLR7/8 antagonists for the treatment of immune disorders.

### SUMMARY OF THE INVENTION

Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present invention relates to novel compounds of formula (I) and (Ia), 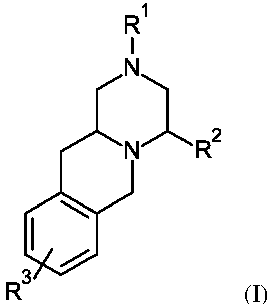 wherein
R¹ is 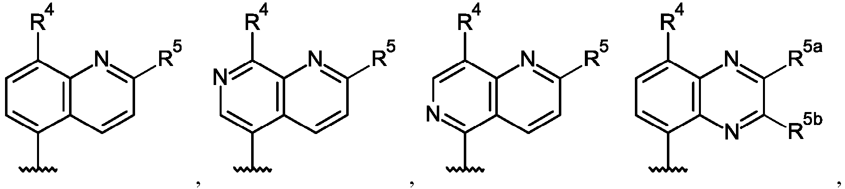 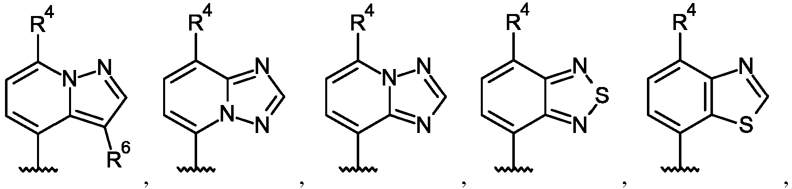 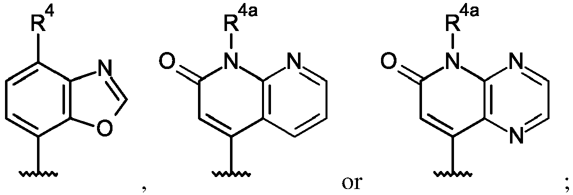 wherein R⁴ is C₁₋₆alkyl, C₁₋₆alkoxy, haloC₁₋₆alkyl, halogen, nitro or cyano; R^{4a} is C₁₋₆alkyl or C₃₋₇cycloalkyl; R⁵, R^{5a} and R^{5b} are independently selected from H and deuterium; R⁶ is H or halogen;
R² is C₁₋₆alkyl;
R³ is unsubstituted or substituted heterocyclyl, heterocyclylamino, heterocyclylC₁₋₆alkylamino or substituted C₁₋₆alkoxy;
or a pharmaceutically acceptable salt thereof.

Another object of the present invention is related to novel compounds of formula (I) or (Ia) or (Ib), their manufacture, medicaments based on a compound in accordance with the invention and their production as well as the use of compounds of formula (I) or (Ia) or (Ib) as TLR7 and/or TLR8 and/or TLR9 antagonist, and for the treatment or prophylaxis of systemic lupus erythematosus or lupus nephritis. The compounds of formula (I) or (Ia) or (Ib) show superior TLR7 and/or TLR8 and/or TLR9 antagonism activity. In addition, the compounds of formula (I) or (Ia) or (Ib) also show good hPBMC, cytotoxicity, solubility, human microsome stability and SDPK profiles, as well as low CYP inhibition.

### DETAILED DESCRIPTION OF THE INVENTION

### DEFINITIONS

The term "C₁₋₆alkyl" denotes a saturated, linear or branched chain alkyl group containing 1 to 6, particularly 1 to 4 carbon atoms, for example methyl, ethyl, n-propyl, isopropyl, n-butyl, isobutyl, *tert-butyl* and the like. Particular "C₁₋₆alkyl" groups are methyl, ethyl and n-propyl.

The term "C₃₋₇cycloalkyl" denotes a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "C₃₋₇cycloalkyl" groups are cyclopropyl, cyclopentyl and cyclohexyl.

The term "halogen" and "halo" are used interchangeably herein and denote fluoro, chloro, bromo, or iodo.

The term "C₁₋₆alkoxy" denotes C₁₋₆alkyl-O-.

The term "halopyrrolidinyl" denotes a pyrrolidinyl substituted once, twice or three times by halogen. Examples of halopyrrolidinyl include, but not limited to, difluoropyrrolidinyl and fluoropyrrolidinyl.

The term "heterocyclyl" or "heterocyclic" denotes a monovalent saturated or partly unsaturated mono or bicyclic ring system of 3 to 12 ring atoms, comprising 1 to 5 ring heteroatoms selected from N, O and S, the remaining ring atoms being carbon. In particular embodiments, heterocyclyl is a monovalent saturated monocyclic ring system of 4 to 7 ring atoms, comprising 1, 2, or 3 ring heteroatoms selected from N, O and S, the remaining ring atoms being carbon. Examples for monocyclic saturated heterocyclyl are aziridinyl, oxiranyl, azetidinyl, oxetanyl, pyrrolidinyl, tetrahydrofuranyl, tetrahydrothienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl. Examples for bicyclic saturated heterocyclic ring are azabicyclo[3.2.1]octyl, quinuclidinyl, oxaazabicyclo[3.2.1]octanyl, azabicyclo[3.3.1]nonanyl, oxaaza-bicyclo[3.3.1]nonanyl, azabicyclo[3.1.0]hexanyl, oxodiazaspiro[3.4]octanyl, acetyloxodiazaspiro[3.4]octanyl, thiaazabicyclo[3.3.1]nonanyl, oxoazaspiro[2.4]heptanyl, oxoazaspiro[3.4]octanyl, oxoazabicyclo[3.1.0]hexanyl and dioxotetrahydropyrrolo[1,2-a]pyrazinyl. Examples for bicyclic heterocyclyl include, but not limited to, 1,2,3,4-tetrahydroisoquinolinyl; 5,6,7,8-tetrahydro-1,6-naphthyridinyl; 5,6,7,8-tetrahydro-1,7-naphthyridinyl; 5,6,7,8-tetrahydro-2,6-naphthyridinyl; 5,6,7,8-tetrahydro-2,7-naphthyridinyl; isoindolinyl; 1-oxo-3a,4,5,6,7,7a-hexahydro-3H-pyrrolo[3,4-c]pyridin-2-yl; 3-oxo-3a,4,5,6,7,7a-hexahydro-1H-pyrrolo[3,4-c]pyridin-2-yl; 3-oxo-1,5,6,7,8,8a-hexahydroimidazo[1,5-a]pyrazin-2-yl. Examples of heterocyclyl can be further substituted by C₁₋₆alkyl, C₃₋₇cycloalkyl, halogen, hydroxy, aminoC₁₋₆alkylcarbonyl, C₁₋₆alkylpyrrolidinylcarbonyl oxo or C₁₋₆alkoxy.

The term "heterocyclylamino" denotes heterocyclyl-NH-.

The term "heterocyclylC₁₋₆alkylamino" denotes heterocyclyl-C₁₋₆alkyl-NH-.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, and polyamine resins.

The term "A pharmaceutically active metabolite" denotes a pharmacologically active product produced through metabolism in the body of a specified compound or salt thereof. After entry into the body, most drugs are substrates for chemical reactions that may change their physical properties and biologic effects. These metabolic conversions, which usually affect the polarity of the compounds of the invention, alter the way in which drugs are distributed in and excreted from the body. However, in some cases, metabolism of a drug is required for therapeutic effect.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### ANTAGONIST OF TLR7 AND/OR TLR8 AND/OR TLR9

The present invention relates to (i) a compound of formula (I), 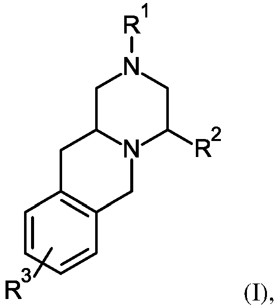 wherein
R¹ is 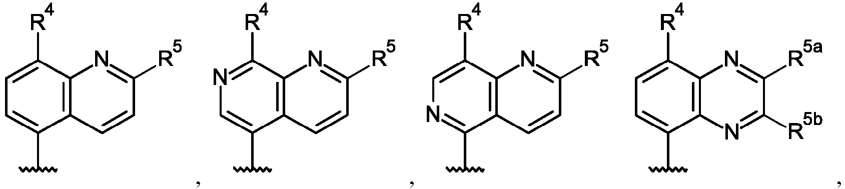 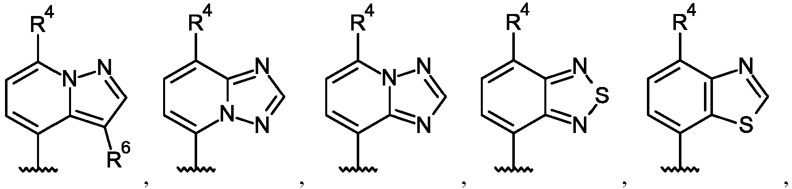 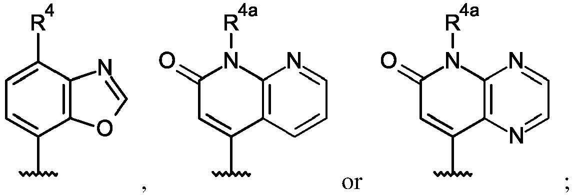 wherein R⁴ is C₁₋₆alkyl, C₁₋₆alkoxy, haloC₁₋₆alkyl, halogen, nitro or cyano; R^{4a} is C₁₋₆alkyl or C₃₋₇cycloalkyl; R⁵, R^{5a} and R^{5b} are independently selected from H and deuterium; R⁶ is H or halogen;
R² is C₁₋₆alkyl;
R³ is unsubstituted or substituted heterocyclyl, heterocyclylamino, heterocyclylC₁₋₆alkylamino or substituted C₁₋₆alkoxy;
   or a pharmaceutically acceptable salt thereof.

A further embodiment of present invention is (ii) a compound of formula (I) according to (i), wherein R¹ is 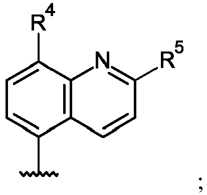 wherein R⁴ is cyano, R⁵ is H or deuterium.

A further embodiment of present invention is (iii) a compound of formula (I) according to (i) or (ii), wherein R³ is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; (hydroxypyrrolidinyl)C₁₋₆alkylamino; (morpholinyl)C₁₋₆alkylamino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; amino-1,4-oxazepanyl; aminohalopyrrolidinyl; aminooxopyrrolidinyl or azetidinylC₁₋₆alkoxy.

Another embodiment of present invention is (iv) a compound of formula (Ia), 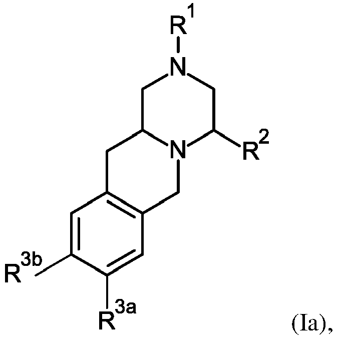 wherein
R¹ is 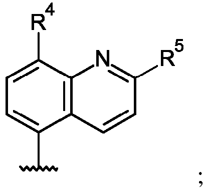 wherein R⁴ is cyano, R⁵ is H or deuterium;
R² is C₁₋₆alkyl;
   a) R^{3a} is (C₁₋₆alkyl)₂aminoC₁₋₆alkoxy; (halopyrrolidinyl)amino; (hydroxypyrrolidinyl)C₁₋₆alkylamino; (morpholinyl)C₁₋₆alkylamino or amino-1,4-oxazepanyl;
      R^{3b} is H; or
   b) R^{3a} is H;
      R^{3b} is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; aminohalopyrrolidinyl; aminooxopyrrolidinyl or azetidinylC₁₋₆alkoxy;
or a pharmaceutically acceptable salt thereof.

Another embodiment of present invention is (v) a compound of formula (Ib), 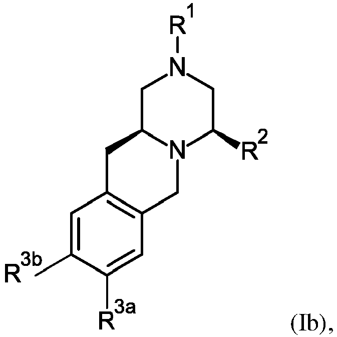 wherein
R¹ is 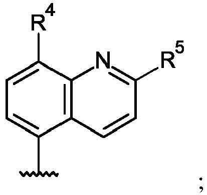 wherein R⁴ is cyano, R⁵ is H or deuterium;
R² is C₁₋₆alkyl;
   a) R^{3a} is (C₁₋₆alkyl)₂aminoC₁₋₆alkoxy; (halopyrrolidinyl)amino; (hydroxypyrrolidinyl)C₁₋₆alkylamino; (morpholinyl)C₁₋₆alkylamino or amino-1,4-oxazepanyl;
      R^{3b} is H; or
   b) R^{3a} is H;
      R^{3b} is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; aminohalopyrrolidinyl; aminooxopyrrolidinyl or azetidinylC₁₋₆alkoxy;
or a pharmaceutically acceptable salt thereof.

A further embodiment of present invention is (vi) a compound of formula (I) or (Ia) or (Ib) according to any one of (i) to (v), wherein
R¹ is 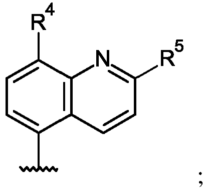 wherein R⁴ is cyano, R⁵ is H or deuterium;
R² is C₁₋₆alkyl;
R^{3a} is H;
R^{3b} is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; aminohalopyrrolidinyl; aminooxopyrrolidinyl or azetidinylC₁₋₆alkoxy;
or a pharmaceutically acceptable salt thereof.

A further embodiment of present invention is (vii) a compound of formula (I) or (Ia) or (Ib) according to any one of (i) to (vi), or a pharmaceutically acceptable salt thereof, wherein R² is methyl.

A further embodiment of present invention is (viii) a compound formula (I) or (Ia) or (Ib) according to any one of (i) to (vii), or a pharmaceutically acceptable salt thereof, wherein R^{3b} is (2-amino-2-methyl-propanoyl)-2,5-diazabicyclo[2.2.1]heptan-2-yl; (2-amino-2-methylpropanoyl)piperazin-1-yl; (2-methylpyrrolidine-2-carbonyl)piperazin-1-yl; (4-fluoropyrrolidin-3-yl)amino; (4-methoxypyrrolidin-3-yl)amino; (azetidin-2-yl)methoxy; 1-oxo-3a,4,5,6,7,7a-hexahydro-3H-pyrrolo[3,4-c]pyridin-2-yl; 2-(dimethylamino)ethoxy; 3,4,4a,5,7,7a-hexahydro-2H-pyrrolo[3,4-b][1,4]oxazin-6-yl; 3-amino-4-fluoro-pyrrolidin-1-yl; 3-amino-4-methoxy-pyrrolidin-1-yl; 3-oxo-1,5,6,7,8,8a-hexahydroimidazo[1,5-a]pyrazin-2-yl; 3-oxo-3a,4,5,6,7,7a-hexahydro-1H-pyrrolo[3,4-c]pyridin-2-yl or 4-amino-2-oxo-pyrrolidin-1-yl.

A further embodiment of present invention is (ix) a compound of formula (I) or (Ia) or (Ib) according to any one of (i) to (viii), or a pharmaceutically acceptable salt thereof, wherein R^{3b} is (C₁₋₆alkylpyrrolidinylcarbonyl)piperazinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl or azetidinylC₁₋₆alkoxy.

A further embodiment of present invention is (x) a compound of formula (I) or (Ia) or (Ib) according to any one of (i) to (ix), wherein R^{3b} is (2-methylpyrrolidine-2-carbonyl)piperazin-1-yl; 3,4,4a,5,7,7a-hexahydro-2H-pyrrolo[3,4-b][1,4]oxazin-6-yl; 3-oxo-1,5,6,7,8,8a-hexahydroimidazo[1,5-a]pyrazin-2-yl or (azetidin-2-yl)methoxy.

A further embodiment of present invention is (xi) a compound of formula (I) or (Ia) or (Ib) according to any one of (i) to (x), wherein
R¹ is 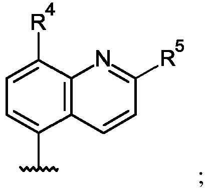 wherein R⁴ is cyano, R⁵ is H or deuterium;
R² is C₁₋₆alkyl;
R^{3a} is H;
R^{3b} is (C₁₋₆alkylpyrrolidinylcarbonyl)piperazinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl or azetidinylC₁₋₆alkoxy;
or a pharmaceutically acceptable salt thereof.

A further embodiment of present invention is (xii) a compound of formula (I) or (Ia) or (Ib) according to any one of (i) to (xi), wherein
R¹ is 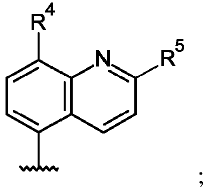 wherein R⁴ is cyano, R⁵ is H or deuterium;
R² is methyl;
R^{3a} is H;
R^{3b} is (2-methylpyrrolidine-2-carbonyl)piperazin-1-yl; 3,4,4a,5,7,7a-hexahydro-2H-pyrrolo[3,4-b][1,4]oxazin-6-yl; 3-oxo-1,5,6,7,8,8a-hexahydroimidazo[1,5-a]pyrazin-2-yl or (azetidin-2-yl)methoxy;
or a pharmaceutically acceptable salt thereof.

Another embodiment of present invention is that (xiii) compounds of formula (I) or (Ia) or (Ib) are selected from the following:
5-[(4*R*,11a*S*)-8-(6-amino-1,4-oxazepan-4-yl)-4-methyl-1,3,4,6,11,11a-hexahydropyrazino[1,2-b]isoquinolin-2-yl]quinoline-8-carbonitrile;
5-[(4*R*,11a*S*)-8-[2-[(3*S*)-3-hydroxypyrrolidin-1-yl]ethylamino]-4-methyl-1,3,4,6,11,11a-hexahydropyrazino[1,2-b]isoquinolin-2-yl]quinoline-8-carbonitrile;
5-[(4*R*,11a*S*)-4-methyl-8-[[(2*R*)-morpholin-2-yl]methylamino]-1,3,4,6,11,11a-hexahydropyrazino[1,2-b]isoquinolin-2-yl]quinoline-8-carbonitrile;
5-[(4*R*,11a*S*)-8-[[(3*S*,4*R*)-4-fluoropyrrolidin-3-yl]amino]-4-methyl-1,3,4,6,11,11a-hexahydropyrazino[1,2-b]isoquinolin-2-yl]quinoline-8-carbonitrile;
5-[(4*R*,11a*S*)-8-[2-(dimethylamino)ethoxy]-4-methyl-1,3,4,6,11,11a-hexahydropyrazino[1,2-*b*]isoquinolin-2-yl]quinoline-8-carbonitrile;
5-[(4*R*,11a*S*)-4-methyl-9-[[(3*R*,4*S*)-4-fluoropyrrolidin-3-yl]amino]-1,3,4,6,11,11a-hexahydropyrazino[1,2-*b*]isoquinolin-2-yl]quinoline-8-carbonitrile;
5-[(4*R*,11a*S*)-9-[[(3*R*,4*S*)-4-methoxypyrrolidin-3-yl]amino]-4-methyl-1,3,4,6,11,11a-hexahydropyrazino[1,2-b]isoquinolin-2-yl]quinoline-8-carbonitrile;
5-[(4*R*,11a*S*)-9-[(3*S*,4*R*)-3-amino-4-fluoro-pyrrolidin-1-yl]-4-methyl-1,3,4,6,11,11a-hexahydropyrazino[1,2-b]isoquinolin-2-yl]quinoline-8-carbonitrile;
5-[(4*R*,11a*S*)-9-[(3*R*,4*S*)-3-amino-4-methoxy-pyrrolidin-1-yl]-4-methyl-1,3,4,6,11,11a-hexahydropyrazino[1,2-b]isoquinolin-2-yl]quinoline-8-carbonitrile;
5-[(4*R*,11a*S*)-9-[(4a*S*,7a*S*)-3,4,4a,5,7,7a-hexahydro-2*H*-pyrrolo[3,4-b][1,4]oxazin-6-yl]-4-methyl-1,3,4,6,11,11a-hexahydropyrazino[1,2-b]isoquinolin-2-yl]quinoline-8-carbonitrile;
5-[(4*R*,11a*S*)-9-[2-(dimethylamino)ethoxy]-4-methyl-1,3,4,6,11,11a-hexahydropyrazino[1,2-b]isoquinolin-2-yl]quinoline-8-carbonitrile;
5-[(4*R*,11a*S*)-9-[[(2*S*)-azetidin-2-yl]methoxy]-4-methyl-1,3,4,6,11,11a-hexahydropyrazino[1,2-b]isoquinolin-2-yl]quinoline-8-carbonitrile;
5-[(4*R*,11a*S*)-4-methyl-9-[[(3*R*,4*S*)-4-fluoropyrrolidin-3-yl]amino]-1,3,4,6,11,11a-hexahydropyrazino[1,2-b]isoquinolin-2-yl]quinoline-8-carbonitrile;
5-[(4*R*,11a*S*)-4-methyl-9-[4-[(2*S*)-2-methylpyrrolidine-2-carbonyl]piperazin-1-yl]-1,3,4,6,11,11a-hexahydropyrazino[1,2-b]isoquinolin-2-yl]quinoline-8-carbonitrile;
5-[(4*R*,11a*S*)-9-[(1*S*,4*S*)-5-(2-amino-2-methyl-propanoyl)-2,5-diazabicyclo[2.2.1]heptan-2-yl]-4-methyl-1,3,4,6,11,11a-hexahydropyrazino[1,2-b]isoquinolin-2-yl]quinoline-8-carbonitrile;
5-[(4*R*,11a*S*)-9-(4-amino-2-oxo-pyrrolidin-1-yl)-4-methyl-1,3,4,6,11,11a-hexahydropyrazino[1,2-b]isoquinolin-2-yl]quinoline-8-carbonitrile;
5-[(4*R*,11a*S*)-4-methyl-9-[(8a*S*)-3-oxo-1,5,6,7,8,8a-hexahydroimidazo[1,5-a]pyrazin-2-yl]-1,3,4,6,11,11a-hexahydropyrazino[1,2-b]isoquinolin-2-yl]quinoline-8-carbonitrile;
5-[(4*R*,11a*S*)-4-methyl-9-[(8a*R*)-3-oxo-1,5,6,7,8,8a-hexahydroimidazo[1,5-a]pyrazin-2-yl]-1,3,4,6,11,11a-hexahydropyrazino[1,2-b]isoquinolin-2-yl]quinoline-8-carbonitrile;
5-[(4*R*,11a*S*)-4-methyl-9-(3-oxo-3a,4,5,6,7,7a-hexahydro-1*H*-pyrrolo[3,4-c]pyridin-2-yl)-1,3,4,6,11,11a-hexahydropyrazino[1,2-b]isoquinolin-2-yl]quinoline-8-carbonitrile; and
5-[(4*R*,11a*S*)-4-methyl-9-(1-oxo-3a,4,5,6,7,7a-hexahydro-3*H*-pyrrolo[3,4-c]pyridin-2-yl)-1,3,4,6,11,11a-hexahydropyrazino[1,2-b]isoquinolin-2-yl]quinoline-8-carbonitrile;
or a pharmaceutically acceptable salt, enantiomer or diastereomer thereof.

### SYNTHESIS

The compounds of the present invention can be prepared by any conventional means. Suitable processes for synthesizing these compounds as well as their starting materials are provided in the schemes below and in the examples. All substituents, in particular, R¹ to R⁶ are as defined above unless otherwise indicated. Furthermore, and unless explicitly otherwise stated, all reactions, reaction conditions, abbreviations and symbols have the meanings well known to a person of ordinary skill in organic chemistry.

A general synthetic route for preparing the compound of formula (I) is shown in Scheme 1 below. 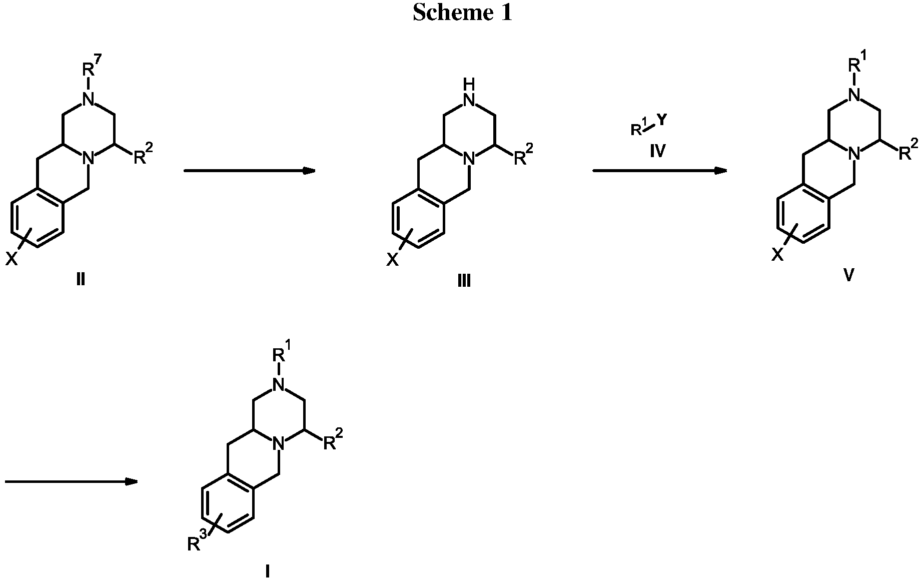 wherein X and Y are halogen or leaving group, for example, OTf or OMs; R⁷ is protecting group, for example, Boc or benzyl.

After R⁷ is removed from compound of formula (II) by selective deprotection, the resulting compound of formula (III) can react with compound of formula (IV) to afford compound of formula (V) by nucleophilic aromatic substitution in the presence of a base, such as DIEPA, or Buchwald-Hartwig amination conditions (*e*.*g*. heating with halide (IV) in the presence of a catalyst, such as Ruphos Pd-G2, and a base, such as Cs₂CO₃. Compound of formula (I) or (Ia) or (Ib) can be obtained from the reaction of compound of formula (V) and R³-H (R³-H can be an amine or an alcohol) via metal catalyzed coupling conditions: Buchwald-Hartwig amination in the presence of a catalyst, such as Ruphos Pd-G2, and a base, such as Cs₂CO_{3;} Suzuki coupling with R³-boronic acid, R³-boronic ester, in the presence of a palladium catalyst, such as tetrakis(triphenylphosphine)palladium(0) or [1,1'-bis(diphenylphosphino)ferrocene]dichloropalladium (II), complex with dichloromethane and a base, such as potassium carbonate in solvent; Stille coupling with organotin reagent, in the presence of a palladium(0) catalyst, such as tetrakis(triphenylphosphine)palladium(0); or Negishi coupling with organozinc reagent in the presence of a palladium(0) catalyst, such as tetrakis(triphenylphosphine)palladium(0) or [1,1'-bis(diphenylphosphino)ferrocene] dichloropalladium (II). In some embodiment, a protecting group, e.g. Boc, will be removed and may be further modified by amide formation reactions before affording the final compound of formula (I) or (Ia) or (Ib).

Compounds of this invention can be obtained as mixtures of diastereomers or enantiomers, which can be separated by methods well known in the art, e.g. (chiral) HPLC or SFC. In another embodiment, compound of formula (Ia) or (Ib) can be obtained according to above scheme by using corresponding chiral starting materials.

Compounds of this invention can be obtained as mixtures of diastereomers or enantiomers, which can be separated by methods well known in the art, e.g. (chiral) HPLC or SFC.

This invention also relates to a process for the preparation of a compound of formula (I), (Ia) comprising the following step:
a) the reaction of compound of formula (V), 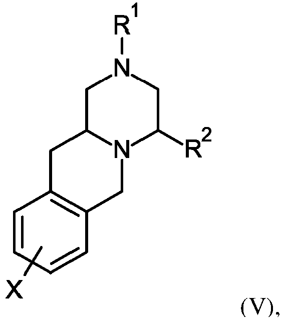 and R³-H via Buchwald-Hartwig amination or Suzuki coupling or Stille coupling or Negishi coupling; wherein R³-H is an amine or an alcohol;

A compound of formula (I) or (Ia) or (Ib) when manufactured according to the above process with achiral or chiral starting materials is also an object of the invention.

### INDICATIONS AND METHODS OF TREATMENT

The present invention provides compounds that can be used as TLR7 and/or TLR8 and/or TLR9 antagonist, which inhibits pathway activation through TLR7 and/or TLR8 and/or TLR9 as well as respective downstream biological events including, but not limited to, innate and adaptive immune responses mediated through the production of all types of cytokines and all forms of auto-antibodies. Accordingly, the compounds of the invention are useful for blocking TLR7 and/or TLR8 and/or TLR9 in all types of cells that express such receptor(s) including, but not limited to, plasmacytoid dendritic cell, B cell, T cell, macrophage, monocyte, neutrophil, keratinocyte, epithelial cell. As such, the compounds can be used as a therapeutic or prophylactic agent for systemic lupus erythematosus and lupus nephritis.

The present invention provides methods for treatment or prophylaxis of systemic lupus erythematosus and lupus nephritis in a patient in need thereof.

Another embodiment includes a method of treating or preventing systemic lupus erythematosus and lupus nephritis in a mammal in need of such treatment, wherein the method comprises administering to said mammal a therapeutically effective amount of a compound of formula (I), a stereoisomer, tautomer, or pharmaceutically acceptable salt thereof.

### EXAMPLES

The invention will be more fully understood by reference to the following examples. They should not, however, be construed as limiting the scope of the invention.

### ABBREVIATIONS

The invention will be more fully understood by reference to the following examples. They should not, however, be construed as limiting the scope of the invention.

Abbreviations used herein are as follows:
- ACN:: acetonitrile
- DCM:: dichloromethane
- EA or EtOAc:: ethyl acetate
- FA:: formic acid
- HATU: 1-[Bis(dimethylamino)methylene]-1H-1,2,3- triazolo[4,5-b]pyridinium 3-oxid hexafluorophosphate
- hr: hour
- hrs: hours
- IC₅₀:: half inhibition concentration
- LCMS: liquid chromatography-mass spectrometry
- MS:: mass spectrometry
- PE:: petroleum ether
- prep-HPLC:: preparative high performance liquid chromatography
- rt:: rt
- RuPhos Pd G2:: chloro(2-dicyclohexylphosphino-2',6'-diisopropoxy-1,1'-biphenyl)[2-(2'-amino-1,1'-biphenyl)]palladium(II) 2nd generation
- SFC:: supercritical fluid chromatography
- TFA:: trifluoroacetic acid
- v/v: volume ratio

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge^{™} Prep-C18 (5 µm, OBDTM 30 × 100 mm) column, SunFire^{™} Prep-C18 (5 µm, OBD^{™} 30 × 100 mm) column, Phenomenex Synergi-C18 (10 µm, 25 × 150 mm) or Phenomenex Gemini-C18 (10 µm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 µm, 30 × 250 mm), AS (10 µm, 30 × 250 mm) or AD (10 µm, 30 × 250 mm) using Mettler Toledo Multigram III system SFC, Waters 80Q preparative SFC or Thar 80 preparative SFC, solvent system: CO₂ and IPA (0.5% TEA in IPA) or CO₂ and MeOH (0.1% NH₃·H₂O in MeOH), back pressure 100bar, detection UV@ 254 or 220 nm.

LC/MS spectra of compounds were obtained using a LC/MS (Waters^{™} Alliance 2795-Micromass ZQ, Shimadzu Alliance 2020-Micromass ZQ or Agilent Alliance 6110-Micromass ZQ), LC/MS conditions were as follows (running time 3 or 1.5 mins):
Acidic condition I: A: 0.1% TFA in H₂O; B: 0.1% TFA in acetonitrile;
Acidic condition II: A: 0.0375% TFA in H₂O; B: 0.01875% TFA in acetonitrile;
Basic condition I: A: 0.1% NH₃·H₂O in H₂O; B: acetonitrile;
Basic condition II: A: 0.025% NH₃·H₂O in H₂O; B: acetonitrile;
Neutral condition: A: H₂O; B: acetonitrile.

Mass spectra (MS): generally only ions which indicate the parent mass are reported, and unless otherwise stated the mass ion quoted is the positive mass ion (MH)⁺.

NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

### PREPARATIVE EXAMPLES

The following examples are intended to illustrate the meaning of the present invention but should by no means represent a limitation within the meaning of the present invention:

### Intermediate A

### 5-Fluoroquinoline-8-carbonitrile

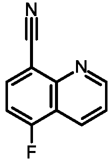

The titled compound was synthesized according to the following scheme:

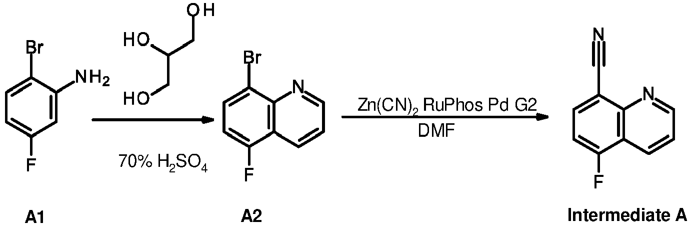

### Step (a): preparation of 8-bromo-5-fluoro-quinoline (compound A2)

In a 100 mL pear-shaped flask, 2-bromo-5-fluoroaniline **(compound A1**, 2.0 g, 10.5 mmol), propane-1,2,3-triol (969 mg, 10.5 mmol) and sodium 3-nitrobenzenesulfonate (2.4 g, 10.5 mmol) were combined with 70% H₂SO₄ (20 mL) to afford a dark brown solution, which was heated to 150 °C and stirred for 3 hrs. After being cooled to room temperature, the reaction mixture was poured into ice-water, and neutralized with sodium hydroxide solution. The resultant mixture was filtered. The filter cake was dissolved in EtOAc and filtered. The resultant filtrate was concentrated *in vacuo.* The crude material was purified by flash chromatography (silica gel, 40 g, 0% to 30% EtOAc in PE) to afford **compound A2** (2.0 g, 84% yield). MS: calc'd 226 and 228 [(M+H)⁺], measured 226 and 228 [(M+H)⁺].

### Step (b): preparation of 5-fluoroquinoline-8-carbonitrile (Intermediate A)

To a solution of 8-bromo-5-fluoroquinoline **(compound A2,** 4.9 g, 21.7 mmol) in DMF (30 mL) was added dicyanozinc (5.0 g, 43.4 mmol) and RuPhos Pd G2 (CAS: 1375325-68-0, Sigma-Aldrich, Catalog: 753246, 842 mg, 1.1 mmol). The reaction mixture was stirred at 100 °C for 3 hrs, then cooled to room temperature. The reaction mixture was filtered and the filtrate was diluted with water (50 ml), then extracted with EA (80 mL) for three times. The combined organic layer was washed with brine, dried over Na₂SO₄, filtered and concentrated *in vacuo.* The residue was purified by flash chromatography (silica gel, 40 g, 0% to 70% EtOAc in PE) to afford **Intermediate A1** (3.0 g, 80 % yield). MS: calc'd 173 [(M+H)⁺], measured 173 [(M+H)⁺]. ¹H NMR (400 MHz, METHANOL-*d*₄) *δ* ppm 9.11 (dd, *J* = 4.28, 1.71 Hz, 1 H), 8.64 (dd, *J =* 8.56, 1.71 Hz, 1 H), 8.29 (dd, *J =* 8.19, 5.62 Hz, 1 H), 7.76 (dd, *J =* 8.56, 4.28 Hz, 1 H), 7.49 (dd, *J =* 9.35, 8.25 Hz, 1 H).

### Intermediate B

### 5-[(4R,11aS)-8-bromo-4-methyl-1,3,4,6,11,11a-hexahydropyrazino[1,2-b]isoquinolin-2-yl] quinoline-8-carbonitrile

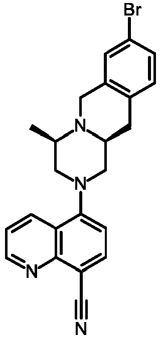

The titled compound was synthesized according to the following scheme:

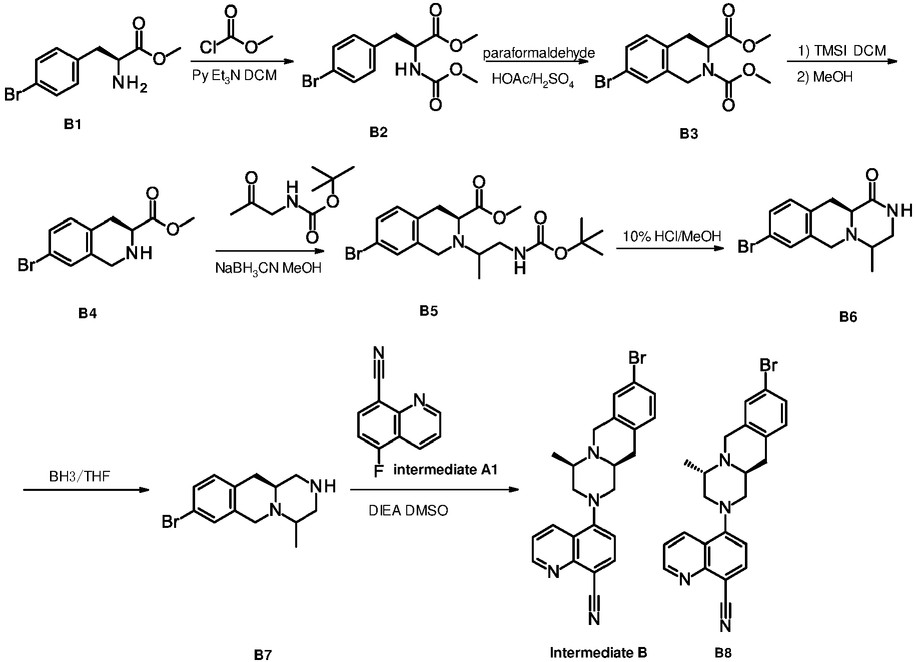

### Step (a): preparation of methyl (2S)-3-(4-bromophenyl)-2-(methoxycarbonylamino) propanoate (compound B2)

To a solution of methyl (S)-2-amino-3-(4-bromophenyl)propanoate hydrochloride (CAS: 880347-43-3, Bidepharm, Catalog: BD00815750) **(compound B1**, 9 g, 30.6 mmol) in DCM (100 mL) was added pyridine (6.0 g, 6.2 mL, 76.4 mmol) and methyl carbonochloridate (5.8 g, 61.1 mmol). The reaction mixture was stirred at room temperature overnight, then the mixture was concentrated under reduced pressure. The residue was dissolved in EtOAc, washed with NaHCO₃ aqueous solution, 1*N* HCl aqueous solution and brine respectively. The organic layer was dried over MgSO₄, filtered, and concentrated under reduced pressure. The residue was purified by flash chromatograph on a silica gel column using 25% Ethyl acetate in hexane to afford the **compound B2** (8.5 g, 88 % yield). MS: calc'd 316 [(M+H)⁺], measured 316 [(M+H)⁺].

### Step (b): preparation of dimethyl (3S)-7-bromo-3,4-dihydro-1H-isoquinoline-2,3 - dicarboxylate (compound B3)

To a mixture of methyl (S)-3-(4-bromophenyl)-2-((methoxycarbonyl)amino)propanoate **(compound B2,** 8.5 g, 26.9 mmol) and paraformaldehyde (1.61 g, 53.8 mmol) was added a mixture of concentrated sulfuric acid (20 mL) and acetic acid (30 mL). The resultant mixture was stirred at room temperature for 20 hrs. The reaction mixture was poured into ice-water, and extracted with DCM. The organic layer was washed with water, saturated NaHCO₃ aqueous solution and brine respectively, dried with MgSO₄. After filtration, the filtrate was concentrated and the residue was purified by flash chromatography (silica gel, 120 g, 0% to 70% EtOAc in hexanes) to afford **compound B3** (5.9 g, 66.9 % yield). MS: calc'd 328 [(M+H)⁺], measured 328[(M+H)⁺].

### Step (c): preparation of methyl (3S)-7-bromo-1,2,3,4-tetrahydroisoquinoline-3-carboxylate (compound B4)

To a solution of dimethyl (S)-7-bromo-3,4-dihydroisoquinoline-2,3(1*H*)-dicarboxylate **(compound B3,** 5.9 g, 18.0 mmol) in DCM (40 mL) was added iodotrimethylsilane (14.4 g, 71.9 mmol). The resultant mixture was stirred at 50 °C for 2 hrs. After being cooled to room temperature, methanol (40 mL) was added, then the reaction mixture was stirred for additional 30 min. The reaction mixture was concentrated in *vacuo,* the residue was diluted with diethyl ether and filtered. The collected solid was washed with ether, dried under vacuum to afford **compound B4** (4.6 g, 94.7 % yield) which was directly used in the next step without further purification. MS: calc'd 270 [(M+H)⁺], measured 270 [(M+H)⁺].

### Step (d): preparation of methyl (3S)-7-bromo-2-[2-(tert-butoxycarbonylamino)-1-methyl-ethyl]-3,4-dihydro-1H-isoquinoline-3-carboxylate (compound B5)

To a solution of methyl (*S*)-7-bromo-1,2,3,4-tetrahydroisoquinoline-3-carboxylate **(compound B4,** 4.6 g, 17 mmol) and *tert*-butyl (2-oxopropyl)carbamate (4.42 g, 25.5 mmol) in methanol (40 mL) was added 0.5 mL HOAc. After the resultant mixture was stirred at reflux for 2 hrs, NaBH₃CN (2.1 g, 34.1 mmol) was added. Then the reaction mixture was stirred at reflux overnight. After the mixture being cooled to room temperature, diluted with water (100 mL), extracted with EA (100 mL) for three times. The combined organic layer was washed with brine, dried over Na₂SO₄, filtered and the filtrate was concentrated. The residue was purified by flash chromatography (silica gel, 80 g, 0% to 70% EtOAc in PE) to afford **compound B5** (3.28 g, 45.1 % yield). MS: calc'd 427 [(M+H)⁺], measured 427[(M+H)⁺].

### Step (e): preparation of (11aS)-8-bromo-4-methyl-2,3,4,6,11,11a-hexahydropyrazino [1,2-b]isoquinolin-1-one (compound B6)

The mixture of methyl (3S)-7-bromo-2-[2-(tert-butoxycarbonylamino)-1-methyl-ethyl]-3,4-dihydro-1H-isoquinoline-3-carboxylate **(compound B5,** 2.2 g, 5.2 mmol) and HCl/MeOH (10% W/W; 50 mL) was stirred at 60 °C overnight. After being cooled to room temperature, the reaction mixture was basified with sat. K₂CO₃ (aq) to pH about 8, and extracted with EA (80 mL) for three times. The combined organic layer was washed with brine (50 mL), dried over Na₂SO₄ and concentrated *in vacuo.* The residue was purified by flash chromatography (silica gel, 40 g, 0% to 20% MeOH in DCM) to afford **compound B6** (780 mg, 51.3 % yield). MS: calc'd 295 [(M+H)⁺], measured 295 [(M+H)⁺].

### Step (f): preparation of (11aS)-8-bromo-4-methyl-2,3,4,6,11,11a-hexahydro-1H-pyrazino[1,2-b]isoquinoline (compound B7)

A mixture of (11aS)-8-bromo-4-methyl-2,3,4,6,11,11a-hexahydropyrazino[1,2-b] isoquinolin-1-one **(compound B6,** 760 mg, 2.57 mmol) and BH₃ solution (1M in THF, 30 mL, 30 mmol) was heated at 80 °C with stirring on for 5 hrs. HCl solution (6 *N,* 5 mL) was added slowly to the reaction mixture at 0 °C. After being stirred at 80 °C for 2 hrs, the mixture was cooled to room temperature, basified with 2 *N* NaOH solution (aq) to pH 10, and extracted with EtOAc twice. The combined organic layer was dried over MgSO₄, filtered and concentrated *in vacuo* to afford **compound B7** (710 mg, 98.1 % yield). MS: calc'd 281 [(M+H)⁺], measured 281 [(M+H)⁺].

### Step (g): preparation of 5-[(4R,11aS)-8-bromo-4-methyl-1,3,4,6,11,11a-hexahydropyrazino [1,2-b]isoquinolin-2-yl]quinoline-8-carbonitrile (Intermediate B)

To a solution of (11a*S*)-8-bromo-4-methyl-2,3,4,6,11,11a-hexahydro-1*H*-pyrazino[1,2-b] isoquinoline **(compound B7,** 710 mg, 2.5 mmol) in DMSO (10 mL) was added 5-fluoroquinoline-8-carbonitrile **(Intermediate A,** 435 mg, 2.5 mmol) and DIEA (1.63 g, 2.2 mL, 12.6 mmol). The resultant mixture was stirred at 120 °C for 20 hrs. After being cooled to room temperature, the reaction was diluted with water (50 mL) and extracted with EA (80 mL) twice. The combined organic layer was washed with brine, dried over Na₂SO₄, filtered and concentrated *in vacuo.* The residue was purified by flash chromatography (silica gel, 40 g, 0% to 100% EtOAc in PE) to afford **Intermediate B** (510 mg, 46.6 % yield) and **compound B8** (230 mg, 21 % yield). The stereochemistry was confirmed by NOESY.

**Intermediate B** MS: calc'd 433 [(M+H)⁺], measured 433 [(M+H)⁺]. ¹H NMR (400 MHz, METHANOL-*d*₄) *δ* ppm 9.00 (dd, *J =* 4.22, 1.65 Hz, 1 H), 8.64 (dd, *J =* 8.56, 1.59 Hz, 1 H), 8.15 (d, *J* = 8.07 Hz, 1 H), 7.65 (dd, *J =* 8.56, 4.28 Hz, 1 H), 7.35 (s, 1 H), 7.31 (dd, *J =* 8.13, 1.90 Hz, 1 H), 7.25 (d, *J* = 7.95 Hz, 1 H), 7.05 (d, *J =* 8.19 Hz, 1H), 4.37 (d, *J =* 15.65 Hz, 1 H), 3.60 (dt, *J =* 11.83, 2.83 Hz, 1 H), 3.46 (br d, *J =* 2.57 Hz, 1 H), 3.45 - 3.37 (m, 1 H), 3.02 - 2.94 (m, 1 H), 2.91 - 2.83 (m, 1 H), 2.88 - 2.82 (m, 1 H), 2.87 - 2.80 (m, 1 H), 2.74 - 2.63 (m, 1 H), 1.37 - 1.29 (m, 3 H).

**compound B8** MS: calc'd 433 [(M+H)⁺], measured 433 [(M+H)⁺]. ¹H NMR (400 MHz, METHANOL-*d*₄) *δ* ppm 8.99 (dd, *J =* 4.22, 1.65 Hz, 1 H), 8.69 (dd, *J =* 8.56, 1.59 Hz, 1 H), 8.17 (d, *J* = 8.07 Hz, 1 H), 7.66 (dd, *J =* 8.56, 4.28 Hz, 1 H), 7.39 - 7.22 (m, 3 H), 7.10 (d, *J =* 8.19 Hz, 1 H), 4.08 - 3.98 (m, 2 H), 3.62 - 3.47 (m, 2 H), 3.39 (dd, *J =* 11.80, 2.02 Hz, 1 H), 3.36 - 3.43 (m, 1 H), 3.28 - 3.17 (m,1 H), 3.16 - 3.09 (m, 1 H), 3.08 - 2.97 (m, 1 H), 2.82 (dd, *J* = 17.06, 4.95 Hz, 1 H), 1.34 - 1.28 (m, 3 H).

### Intermediate C

### 5-[(4R,11aS)-9-bromo-4-methyl-1,3,4,6,11,11a-hexahydropyrazino[1,2-b]isoquinolin-2-yl] quinoline-8-carbonitrile

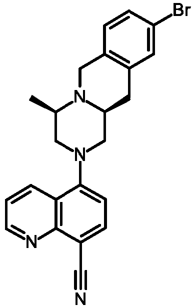

The title compound was prepared in analogy to the preparation of **Intermediate B** by using methyl (*S*)-2-amino-3-(3-bromophenyl)propanoate hydrochloride (CAS: 875782-96-0, Bidepharm, Catalog: BD00869561) instead of methyl (S)-2-amino-3-(4-bromophenyl)propanoate hydrochloride in step (a). **Intermediate C** was obtained. MS: calc'd 433 [(M+H)⁺], measured 433 [(M+H)⁺]. ¹H NMR (CHLOROFORM-d, 400 MHz) δ 9.08 (dd, 1H, J=1.6, 4.2 Hz), 8.49 (dd, 1H, J=1.7, 8.6 Hz), 8.05 (d, 1H, J=7.9 Hz), 7.52 (dd, 1H, J=4.2, 8.6 Hz), 7.29 (d, 1H, J=8.2 Hz), 7.26 (br s, 1H), 7.11 (d, 1H, J=8.1 Hz), 6.98 (d, 1H, J=8.2 Hz), 4.30 (d, 1H, J=15.2 Hz), 3.5-3.6 (m, 1H), 3.3-3.4 (m, 2H), 2.7-3.0 (m, 5H), 1.29 (d, 3H, J=6.1 Hz). The stereochemistry was confirmed by NOESY.

### Example 1

### 5-[(4R,11aS)-8-(6-amino-1,4-oxazepan-4-yl)-4-methyl-1,3,4,6,11,11a-hexahydropyrazino[1,2-b]isoquinolin-2-yl]quinoline-8-carbonitrile

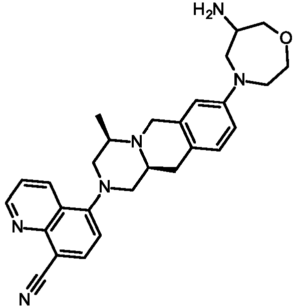

The titled compound was synthesized according to the following scheme:

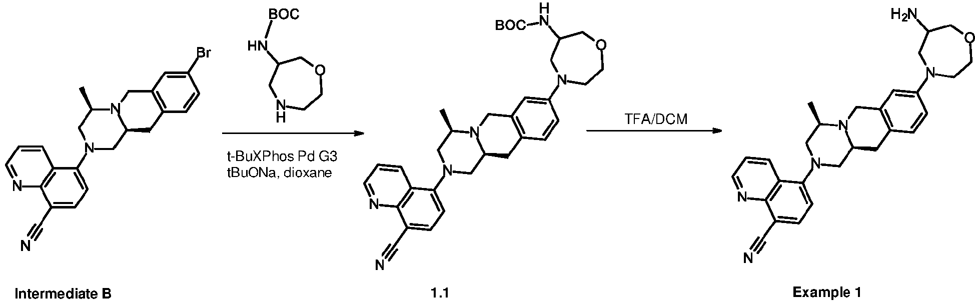

### Step (a): preparation of tert-butyl N-[4-[(4R,11aS)-2-(8-cyano-5-quinolyl)-4-methyl-1,3,4,6,11,11a-hexahydropyrazino[1,2-b]isoquinolin-8-yl]-1,4-oxazepan-6-yl]carbamate (compound 1.1)

To a solution of *tert*-butyl *N*-(1,4-oxazepan-6-yl)carbamate (CAS: 1782916-90-8, PharmaBlock, Catalog: PB95734, 60 mg, 277 µmol) in dioxane (5 mL) was added 5-((4*R*,11a*S*)-8-bromo-4-methyl-1,3,4,6,11,11a-hexahydro-2*H*-pyrazino[1,2-b]isoquinolin-2-yl)quinoline-8-carbonitrile **(Intermediate B1**, 120 mg, 277 µmol), *t*BuONa (53.3 mg, 555 µmol) and tBuXPhos Pd G3 (CAS: 1447963-75-8, Sigma-Aldrich, Catalog: 762229, 22 mg, 27.7 µmol). The resultant mixture was stirred at 90 °C overnight. After being cooled to room temperature, diluted with water (20 mL), and extracted with EA (20 mL) for three times. The combined organic layer was washed with brine, dried over Na₂SO₄, filtered and concentrated *in vacuo.* The residue was purified by flash chromatography (silica gel, 24 g, 0% to 100% EtOAc in PE) to afford **compound 1.1** (33 mg, 20.9 % yield). MS: calc'd 569 [(M+H)⁺], measured 569 [(M+H)⁺].

### Step (b): preparation of 5-[(4R,11aS)-8-(6-amino-1,4-oxazepan-4-yl)-4-methyl-1,3, 4,6,11,11a-hexahydropyrazino[1,2-b]isoquinolin-2-yl]quinoline-8-carbonitrile (Example 1)

To a solution of *tert*-butyl *N*-[4-[(4*R*,11a*S*)-2-(8-cyano-5-quinolyl)-4-methyl-1,3,4,6,11,11a-hexahydropyrazino[1,2-b]isoquinolin-8-yl]-1,4-oxazepan-6-yl]carbamate **(compound 1.1, 21** mg, 38 µmol ) in DCM (2 mL) was added TFA (1 mL). The reaction mixture was stirred at room temperature for 30 min, then concentrated to afford a crude product, which was purified by prep-HPLC to afford **Example** 1 (9 mg, 50.6% yield). MS: calc'd 469 [(M+H)⁺], measured 469 [(M+H)⁺]. ¹H NMR (400 MHz, METHANOL-*d*₄) *δ* ppm 9.09 - 8.96 (m, 1H), 8.76 - 8.60 (m, 1H), 8.20 (d, *J* = 7.9 Hz, 1H), 7.74 - 7.64 (m, 1H), 7.35 (d, *J* = 7.9 Hz, 1H), 7.10 (d, *J =* 8.6 Hz, 1H), 6.88 - 6.80 (m, 1H), 6.74 (s, 1H), 4.67 (d, *J =* 15.3 Hz, 1H), 4.18 - 4.10 (m, 1H), 4.07 - 3.75 (m, 7H), 3.69 - 3.43 (m, 6H), 3.12 - 2.97 (m, 3H), 2.87 (br d, *J =* 11.9 Hz, 1H), 1.49 (d, *J =* 6.2 Hz, 3H).

### Example 2

### 5-[(4R,11aS)-8-[2-[(3S)-3-hydroxypyrrolidin-1-yl]ethylamino]-4-methyl-1,3,4,6,11,11a-hexahydropyrazino[1,2-b]isoquinolin-2-yl]quinoline-8-carbonitrile

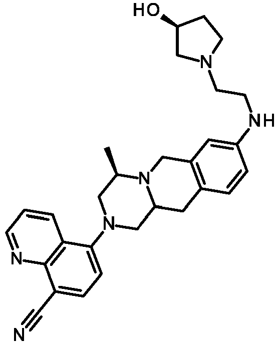

The title compound was prepared in analogy to the preparation of **Example 1** by using (3S)-1-(2-aminoethyl)pyrrolidin-3-ol (CAS: 540787-75-5, Bide Pharmatech, Catalog: BD45313) instead of *tert*-butyl *N*-(1,4-oxazepan-6-yl)carbamate in step (a). **Example 2** was obtained. MS: calc'd 483 [(M+H)⁺], measured 483 [(M+H)⁺]. ¹H NMR (400 MHz, METHANOL-*d*₄) *δ* ppm 9.05 - 8.98 (m, 1H), 8.73 - 8.64 (m, 1H), 8.20 (d, *J =* 7.9 Hz, 1H), 7.72 - 7.63 (m, 1H), 7.35 (d, *J* = 8.1 Hz, 1H), 7.03 (d, *J =* 8.3 Hz, 1H), 6.83 - 6.63 (m, 1H), 6.58 (d, *J =* 2.2 Hz, 1H), 4.67 (d, *J* = 15.4 Hz, 1H), 4.63 - 4.56 (m, 1H), 4.00 (d, *J =* 15.2 Hz, 1H), 3.83 - 3.74 (m, 1H), 3.72 - 3.60 (m, 3H), 3.59 - 3.40 (m, 8H), 3.14 - 2.94 (m, 3H), 2.88 (br d, *J =* 11.5 Hz, 1H), 2.35 - 2.19 (m, 1H), 2.15 - 2.02 (m, 1H), 1.50 (d, *J =* 6.4 Hz, 3H).

### Example 3

### 5-[(4R,11aS)-4-methyl-8-[[(2R)-morpholin-2-yl]methylamino]-1,3,4,6,11,11a-hexahydropyrazino[1,2-b]isoquinolin-2-yl]quinoline-8-carbonitrile

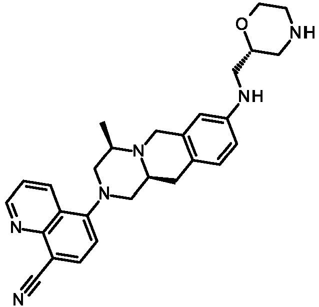

The title compound was prepared in analogy to the preparation of **Example** 1 by using *tert-*butyl (2S)-2-(aminomethyl)morpholine-4-carboxylate (CAS: 879403-42-6, PharmaBlock, Catalog: PBN20121306) instead of *tert-butyl N*-(1,4-oxazepan-6-yl)carbamate in step (a). **Example** 3 was obtained. MS: calc'd 469 [(M+H)⁺], measured 469 [(M+H)⁺]. ¹H NMR (400 MHz, METHANOL-*d*₄) *δ* ppm 9.09 - 8.97 (m, 1H), 8.77 - 8.64 (m, 1H), 8.21 (d, *J* = 7.9 Hz, 1H), 7.79 - 7.58 (m, 1H), 7.39 (d, *J =* 8.1 Hz, 1H), 7.03 (d, *J =* 8.4 Hz, 1H), 6.75 - 6.64 (m, 1H), 6.57 (d, *J =* 2.2 Hz, 1H), 4.87 (br d, *J =* 15.5 Hz, 1H), 4.36 (d, *J =* 15.4 Hz, 1H), 4.18 - 4.05 (m, 2H), 4.02 - 3.70 (m, 5H), 3.43 (br d, *J =* 12.5 Hz, 1H), 3.32 - 2.90 (m, 9H), 1.62 (d, *J =* 6.5 Hz, 3H)

### Example 4

### 5-[(4R,11aS)-8-[[(3S,4R)-4-fluoropyrrolidin-3-yl]amino]-4-methyl-1,3,4,6,11,11a-hexahydropyrazino[1,2-b]isoquinolin-2-yl]quinoline-8-carbonitrile

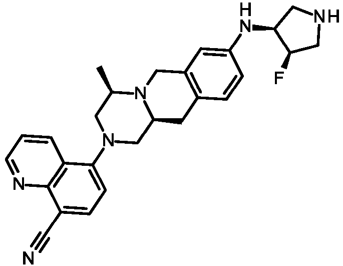

The title compound was prepared in analogy to the preparation of **Example 1** by using *tert-*butyl (3*S*,4*R*)-3-amino-4-fluoro-pyrrolidine-1-carboxylate (CAS: 1174020-30-4, PharmaBlock, Catalog: PB07374) instead of *tert*-butyl *N*-(1,4-oxazepan-6-yl)carbamate in step (a). **Example 4** was obtained. MS: calc'd 457 [(M+H)⁺], measured 457 [(M+H)⁺]. ¹H NMR (400 MHz, METHANOL-*d*₄) *δ* ppm 9.09 - 8.98 (m, 1H), 8.74 - 8.65 (m, 1H), 8.19 (d, *J =* 7.9 Hz, 1H), 7.72 - 7.65 (m, 1H), 7.34 (d, *J =* 8.1 Hz, 1H), 7.03 (d, *J =* 8.4 Hz, 1H), 6.79 - 6.71 (m, 1H), 6.65 (d, *J* = 2.0 Hz, 1H), 5.44 - 5.22 (m, 1H), 4.61 (d, *J =* 15.2 Hz, 1H), 4.52 - 4.38 (m, 1H), 3.92 (br d, *J =* 15.2 Hz, 1H), 3.84 - 3.37 (m, 7H), 3.24 (t, *J =* 11.2 Hz, 1H), 3.12 - 2.94 (m, 3H), 2.86 (br d, *J =* 11.7 Hz, 1H), 1.47 (d, *J =* 6.2 Hz, 3H).

### Example 5

### 5-[(4R,11aS)-8-[2-(dimethylamino)ethoxy]-4-methyl-1,3,4,6,11,11a-hexahydropyrazino[1,2-b]isoquinolin-2-yl]quinoline-8-carbonitrile

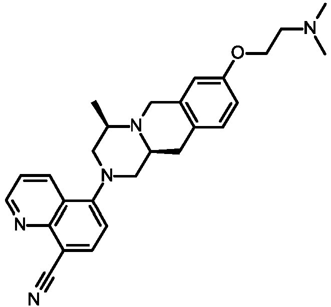

The title compound was prepared in analogy to the preparation of **Example 1** by using 2-(dimethylamino)ethanol (CAS: 108-01-0, Sigma-Aldrich, Catalog: 38990) instead of *tert-butyl N*-(1,4-oxazepan-6-yl)carbamate in step (a). **Example 5** was obtained. MS: calc'd 442 [(M+H)⁺], measured 442 [(M+H)⁺]. ¹H NMR (400 MHz, METHANOL-*d*₄) *δ* ppm 8.89 - 8.81 (m, 1H), 8.60 - 8.45 (m, 1H), 8.04 (d, *J* = 7.9 Hz, 1H), 7.60 - 7.47 (m, 1H), 7.17 (d, *J =* 8.1 Hz, 1H), 7.00 (d, *J* = 8.6 Hz, 1H), 6.81 - 6.71 (m, 2H), 4.42 (d, *J =* 15.5 Hz, 1H), 4.24 - 4.15 (m, 2H), 3.68 - 3.53 (m, 2H), 3.50 - 3.38 (m, 3H), 3.12 - 3.02 (m, 1H), 2.90 - 2.76 (m, 4H), 2.86 (s, 6H), 2.72 - 2.61 (m, 1H), 1.27 (d, *J =* 6.4 Hz, 3H).

### Example 6

### 5-[(4R,11aS)-4-methyl-9-[[(3R,4S)-4-fluoropyrrolidin-3-yl]amino]-1,3,4,6,11,11a-hexahydropyrazino[1,2-b]isoquinolin-2-yl]quinoline-8-carbonitrile

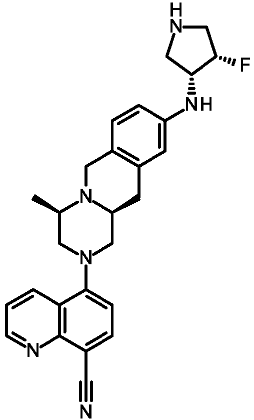

The title compound was prepared in analogy to the preparation of **Example 1** by using *tert-*butyl (3*R*,4*S*)-3-amino-4-fluoro-pyrrolidine-1-carboxylate (CAS: 1009075-48-2, PharmaBlock, Catalog: PB07375) instead of *tert*-butyl *N*-(1,4-oxazepan-6-yl)carbamate and 5-[(4R,11aS)-9-bromo-1,1,1,1,1,4-hexamethyl-4,6,11,11a-tetrahydro-3*H*-pyrazino[1,2-b]isoquinolin-2-yl]quinoline-8-carbonitrile **(Intermediate** C) instead of 5-[(4R,11aS)-8-bromo-4-methyl-1,3,4,6,11,11a-hexahydropyrazino[1,2-b]isoquinolin-2-yl]quinoline-8-carbonitrile **(Intermediate B)** in step (a). **Example 6** was obtained. MS: calc'd 457 [(M+H)⁺], measured 457 [(M+H)⁺]. ¹H NMR (METHANOL-d₄, 400 MHz) *δ* 8.92 (dd, 1H, *J*=1.5, 4.3 Hz), 8.60 (dd, 1H, *J=1.5,* 8.6 Hz), 8.10 (d, 1H, *J*=7.9 Hz), 7.59 (dd, 1H, *J*=4.3, 8.6 Hz), 7.29 (d, 1H, *J*=7.9 Hz), 7.02 (d, 1H, *J*=8.6 Hz), 6.66 (dd, 1H, *J*=2.1, 8.4 Hz), 6.54 (d, 1H, *J*=1.8 Hz), 5.1-5.4 (m, 1H), 4.80 (br d, 1H, *J*=15.0 Hz), 4.3-4.4 (m, 1H), 4.22 (br d, 1H, *J*=15.0 Hz), 4.04 (br t, 1H, *J*=10.9 Hz), 3.8-3.9 (m, 2H), 3.4-3.8 (m, 4H), 3.0-3.2 (m, 4H), 2.8-3.0 (m, 1H), 1.50 (d, 3H, J=6.5 Hz).

### Example 7

### 5-[(4R,11aS)-9-[[(3R,4S)-4-methoxypyrrolidin-3-yl]amino]-4-methyl-1,3,4,6,11,11a-hexahydropyrazino[1,2-b]isoquinolin-2-yl]quinoline-8-carbonitrile

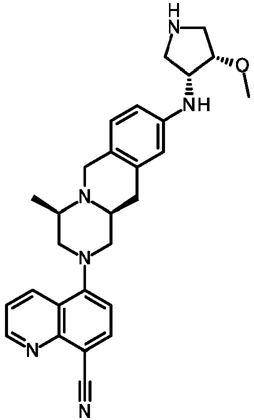

The title compound was prepared in analogy to the preparation of **Example 6** by using *tert-*butyl (3*R*,*4S)*-3-amino-4-methoxy-pyrrolidine-1-carboxylate (CAS: 148260-94-0, BePharm, Catalog: BD285562) instead of *tert*-butyl (3*R*,4*S*)-3-amino-4-fluoro-pyrrolidine-1-carboxylate in step (a). **Example 7** was obtained. MS: calc'd 469 [(M+H)⁺], measured 469 [(M+H)⁺]. ¹H NMR (METHANOL-d₄, 400 MHz) δ 9.06 (dd, 1H, *J*=1.6, 4.3 Hz), 8.73 (dd, 1H, *J*=1.6, 8.6 Hz), 8.25 (d, 1H, *J*=7.9 Hz), 7.72 (dd, 1H, *J*=4.3, 8.6 Hz), 7.42 (d, 1H, *J*=7.9 Hz), 7.14 (d, 1H, *J*=8.4 Hz), 6.77 (dd, 1H, *J*=2.3, 8.4 Hz), 6.64 (d, 1H, *J*=2.0 Hz), 4.93 (br d, 1H, *J*=14.1 Hz), 4.3-4.4 (m, 2H), 4.1-4.2 (m, 2H), 4.0-4.0 (m, 1H), 3.9-4.0 (m, 1H), 3.8-3.9 (m, 1H), 3.6-3.7 (m, 2H), 3.42 (s, 3H), 3.1-3.3 (m, 5H), 3.0-3.1 (m, 1H), 1.62 (d, 3H, *J*=6.5 Hz)

### Example 8

### 5-[(4R,11aS)-9-[(3S,4R)-3-amino-4-fluoro-pyrrolidin-1-yl]-4-methyl-1,3,4,6,11,11a-hexahydropyrazino[1,2-b]isoquinolin-2-yl]quinoline-8-carbonitrile

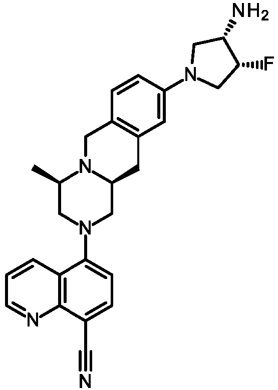

The title compound was prepared in analogy to the preparation of **Example** 6 by using *tert-*butyl N-[(3S,4R)-4-fluoropyrrolidin-3-yl]carbamate (CAS: 1033718-89-6, PharmaBlock, Catalog: PB09206) instead of *tert-butyl* (3*R*,4*S*)-3-amino-4-fluoro-pyrrolidine-1-carboxylate in step (a). **Example** 8 was obtained. MS: calc'd 457 [(M+H)⁺], measured 457 [(M+H)⁺]. ¹H NMR (METHANOL-d₄, 400 MHz) *δ* ppm 9.06 (dd, 1H, J=1.6, 4.3 Hz), 8.73 (dd, 1H, J=1.7, 8.6 Hz), 8.24 (d, 1H, J=7.9 Hz), 7.72 (dd, 1H, J=4.2, 8.5 Hz), 7.41 (d, 1H, J=8.1 Hz), 7.21 (d, 1H, J=8.7 Hz), 6.66 (dd, 1H, J=2.3, 8.3 Hz), 6.51 (d, 1H, J=1.7 Hz), 5.3-5.6 (m, 1H), 4.0-4.4 (m, 3H), 3.7-4.0 (m, 6H), 3.4-3.5 (m, 3H), 3.1-3.3 (m, 2H), 3.0-3.1 (m, 1H), 1.59 (br d, 3H, J=6.5 Hz)

### Example 9

### 5-[(4R,11aS)-9-[(3R,4S)-3-amino-4-methoxy-pyrrolidin-1-yl]-4-methyl-1,3,4,6,11,11a-hexahydropyrazino[1,2-b]isoquinolin-2-yl]quinoline-8-carbonitrile

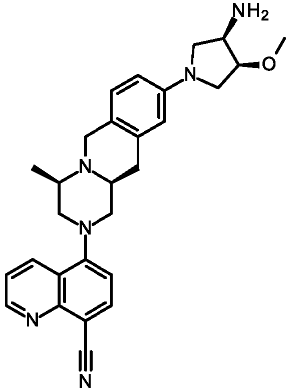

The title compound was prepared in analogy to the preparation of **Example 6** by using *tert-*butyl *N*-[(3*R*,4*S*)-4-methoxypyrrolidin-3-yl]carbamate (CAS: 1932508-77-4, PharmaBlock, Catalog: PBZ4729) instead of *tert*-butyl (3*R*,4*S*)-3-amino-4-fluoro-pyrrolidine-1-carboxylate in step (a). **Example 9** was obtained. MS: calc'd 469 [(M+H)⁺], measured 469 [(M+H)⁺]. ¹H NMR (METHANOL-d₄, 400 MHz) δ 9.06 (dd, 1H, *J*=1.6, 4.3 Hz), 8.73 (dd, 1H, *J*=1.6, 8.6 Hz), 8.24 (d, 1H, *J*=7.9 Hz), 7.72 (dd, 1H, *J*=4.3, 8.6 Hz), 7.41 (d, 1H, *J*=7.9 Hz), 7.19 (d, 1H, *J*=8.4 Hz), 6.64 (dd, 1H, *J*=1.8, 8.8 Hz), 6.49 (d, 1H, *J*=1.6 Hz), 4.7-4.8 (m, 1H), 4.2-4.3 (m, 1H), 4.1-4.2 (m, 1H), 4.0-4.1 (m, 1H), 3.9-4.0 (m, 1H), 3.8-3.9 (m, 1H), 3.8-3.8 (m, 1H), 3.6-3.7 (m, 1H), 3.5-3.6 (m, 1H), 3.4-3.5 (m, 2H), 3.4-3.4 (m, 3H), 3.32 (dd, 1H, *J*=6.7, 9.8 Hz), 3.0-3.2 (m, 3H), 2.9-3.0 (m, 1H), 1.4-1.6 (m, 3H)

### Example 10

### 5-[(4R,11aS)-9-[(4aS,7aS)-3,4,4a,5,7,7a-hexahydro-2H-pyrrolo[3,4-b][1,4]oxazin-6-yl]-4-methyl-1,3,4,6,11,11a-hexahydropyrazino[1,2-b]isoquinolin-2-yl]quinoline-8-carbonitrile

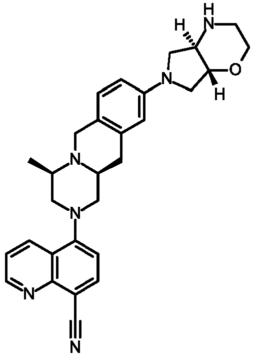

The title compound was prepared in analogy to the preparation of **Example 6** by using *tert-*butyl (4a*R*,7a*R*)-3,4a,5,6,7,7a-hexahydro-2*H*-pyrrolo[3,4-b][1,4]oxazine-4-carboxylate (CAS: 1932337-68-2, PharmaBlock, Catalog: PBXA8123) instead of *tert*-butyl (3*R*,*4*S)-3-amino-4-fluoro-pyrrolidine-1-carboxylate in step (a). **Example 10** was obtained. MS: calc'd 481 [(M+H)⁺], measured 481 [(M+H)⁺]. ¹H NMR (METHANOL-d₄, 400 MHz) δ 9.06 (dd, 1H, *J*=1.5, 4.2 Hz), 8.73 (dd, 1H, *J*=1.7, 8.6 Hz), 8.25 (d, 1H, *J*=7.9 Hz), 7.72 (dd, 1H, *J*=4.3, 8.7 Hz), 7.42 (d, 1H, *J*=7.9 Hz), 7.20 (d, 1H, *J*=8.6 Hz), 6.64 (dd, 1H, *J*=2.2, 8.8 Hz), 6.48 (d, 1H, *J*=2.4 Hz), 4.90 (br d, 1H, *J=15.0* Hz), 4.36 (br d, 1H, *J=15.0* Hz), 4.28 (dd, 1H, J=3.9, 13.4 Hz), 3.9-4.2 (m, 5H), 3.7-3.9 (m, 3H), 3.4-3.7 (m, 4H), 3.0-3.3 (m, 5H), 1.62 (d, 3H, J=6.5 Hz)

### Example 11

### 5-[(4R,11aS)-9-[2-(dimethylamino)ethoxy]-4-methyl-1,3,4,6,11,11a-hexahydropyrazino[1,2-b]isoquinolin-2-yl]quinoline-8-carbonitrile

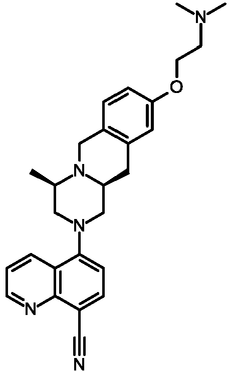

The title compound was prepared in analogy to the preparation of **Example 6** by using 2-(dimethylamino)ethanol instead of *tert*-butyl (3*R*,4*S*)-3-amino-4-fluoro-pyrrolidine-1-carboxylate in step (a). **Example 11** was obtained. MS: calc'd 442 [(M+H)⁺], measured 442 [(M+H)⁺]. ¹H NMR (METHANOL-d₄, 400 MHz) δ 9.03 (dd, 1H, *J*=1.5, 4.3 Hz), 8.72 (dd, 1H, *J*=1.5, 8.7 Hz), 8.22 (d, 1H, *J*=7.9 Hz), 7.71 (dd, 1H, *J*=4.2, 8.6 Hz), 7.41 (d, 1H, *J*=7.9 Hz), 7.31 (d, 1H, *J*=8.7 Hz), 7.04 (dd, 1H, *J*=2.4, 8.6 Hz), 6.96 (d, 1H, *J*=2.2 Hz), 5.00 (br d, 1H, *J*=15.3 Hz), 4.3-4.5 (m, 3H), 4.19 (br t, 1H, *J*=10.3 Hz), 3.9-4.1 (m, 2H), 3.80 (br d, 1H, J=13.3 Hz), 3.5-3.7 (m, 2H), 3.2-3.3 (m, 3H), 3.13 (br dd, 1H, *J*=11.9, 17.7 Hz), 3.01 (s, 6H), 1.64 (d, 3H, *J*=6.5 Hz)

### Example 12

### 5-[(4R,11aS)-9-[[(2S)-azetidin-2-yl]methoxy]-4-methyl-1,3,4,6,11,11a-hexahydropyrazino[1,2-b]isoquinolin-2-yl]quinoline-8-carbonitrile

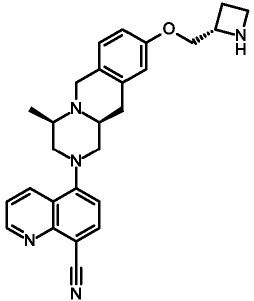

The title compound was prepared in analogy to the preparation of **Example 6** by using *tert-*butyl (2*S*)-2-(hydroxymethyl)azetidine-1-carboxylate (CAS: 161511-85-9, PharmaBlock, Catalog: PB03034) instead of *tert*-butyl (3*R*,4*S*)-3-amino-4-fluoro-pyrrolidine-1-carboxylate in step (a). **Example 12** was obtained. MS: calc'd 440 [(M+H)⁺], measured 440 [(M+H)⁺]. ¹H NMR (METHANOL-d₄, 400 MHz) δ 9.06 (dd, 1H, *J*=1.7, 4.2 Hz), 8.73 (dd, 1H, *J*=1.6, 8.6 Hz), 8.25 (d, 1H, *J*=7.9 Hz), 7.73 (dd, 1H, *J*=4.3, 8.6 Hz), 7.43 (d, 1H, *J*=8.1 Hz), 7.33 (d, 1H, J=8.7 Hz), 7.07 (dd, 1H, *J*=2.4, 8.5 Hz), 6.98 (d, 1H, *J*=2.3 Hz), 5.01 (br d, 1H, *J*=15.0 Hz), 4.41 (br d, 1H, *J*=15.0 Hz), 4.35 (d, 2H, *J*=4.6 Hz), 3.9-4.3 (m, 6H), 3.8-3.9 (m, 1H), 3.1-3.3 (m, 4H), 2.6-2.8 (m, 2H), 1.63 (d, 3H, *J*=6.5 Hz).

### Example 13

### 5-[(4R,11aS)-4-methyl-9-[[(3R,4S)-4-fluoropyrrolidin-3-yl]amino]-1,3,4,6,11,11a-hexahydropyrazino[1,2-b]isoquinolin-2-yl]quinoline-8-carbonitrile

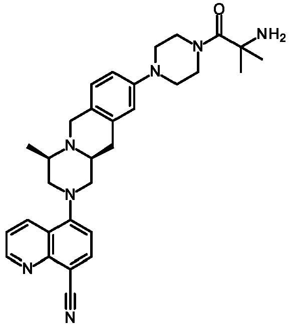

The titled compound was synthesized according to the following scheme:

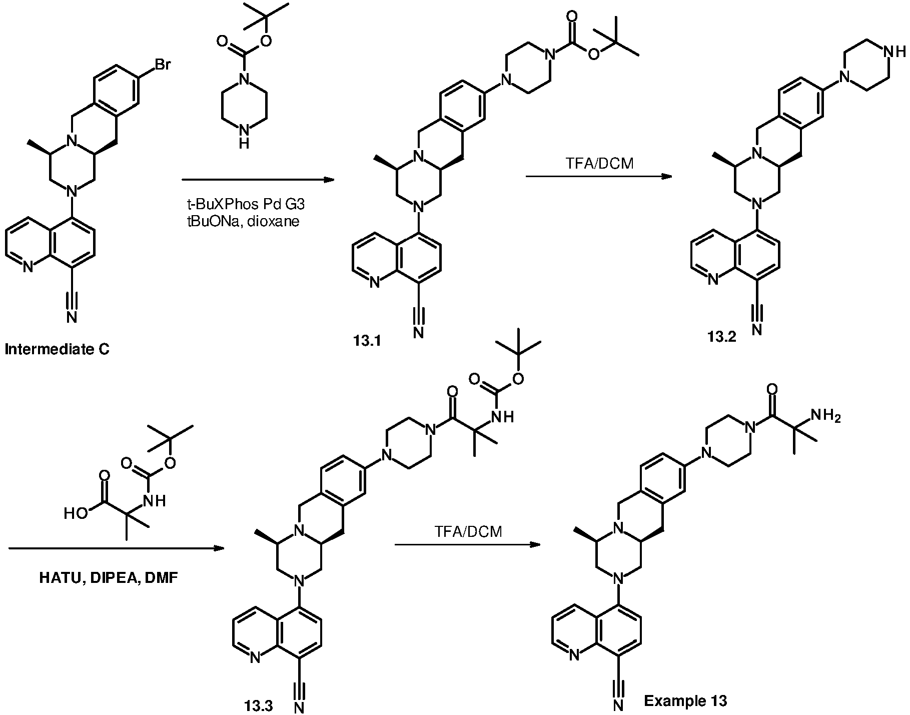

### Step (a): preparation of tert-butyl 4-[(4R,11aS)-2-(8-cyano-5-quinolyl)-4-methyl-1,3,4,6,11,11a-hexahydropyrazino[1,2-b]isoquinolin-9-yl]piperazine-1-carboxylate (compound 13.1)

To a solution of *tert-*butyl piperazine-1-carboxylate (60 mg, 322 µmol) in dioxane (5 mL) was added 5-((4R,11aS)-9-bromo-4-methyl-1,3,4,6,11,11a-hexahydro-2*H*-pyrazino[1,2-b]isoquinolin-2-yl) quinoline-8-carbonitrile **(Intermediate C,** 139.4 mg, 277 µmol), tBuONa (61.9 mg, 644 µmol) and tBuXPhos Pd G3 (CAS: 1447963-75-8, Sigma-Aldrich, Catalog: 762229, 25.6 mg, 32.2 µmol). The resultant mixture was stirred at 90 °C overnight. After being cooled to room temperature, the mixture was diluted with water (20 mL), and extracted with EA (20 mL) for three times. The combined organic layer was washed with brine, dried over Na₂SO₄, filtered and concentrated *in vacuo.* The residue was purified by flash chromatography (silica gel, 24 g, 0% to 100% EtOAc in PE) to afford **compound 13.1** (26.1 mg, 35 % yield). MS: calc'd 539 [(M+H)⁺], measured 539 [(M+H)⁺].

### Step (b): preparation of 5-[(4R,11aS)-4-methyl-9-piperazin-1-yl-1,3,4,6,11,11a-hexahydropyrazino[1,2-b]isoquinolin-2-yl]quinoline-8-carbonitrile (compound 13.2)

To a solution of *tert*-butyl 4-[(4*R*,11a*S*)-2-(8-cyano-5-quinolyl)-4-methyl-1,3,4,6,11,11a-hexahydropyrazino[1,2-b]isoquinolin-9-yl]piperazine-1-carboxylate **(compound 13.1,** 26.1 mg, 48.5 µmol ) in DCM (2 mL) was added TFA (1 mL). The reaction mixture was stirred at room temperature for 30 min, quenched with saturated aq.NaHCO₃, washed with brine and dried over Na₂SO₄, then concentrated to afford **compound 13.2** (20.2 mg, 95% yield). MS: calc'd 439 [(M+H)⁺], measured 439 [(M+H)⁺].

### Step (c): preparation of tert-butyl N-[1,1-dimethyl-2-oxo-2-[4-[(4R,11aS)-2-(8-cyano-5-quinolyl)-4-methyl-1,3,4,6,11,11a-hexahydropyrazino[1,2-b]isoquinolin-9-yl]piperazin-1-yl]ethyl]carbamate (compound 13.3)

To a solution of 5-[(4*R*,11a*S*)-4-methyl-9-piperazin-1-yl-1,3,4,6,11,11a-hexahydropyrazino[1,2-b]isoquinolin-2-yl]quinoline-8-carbonitrile **(compound 13.2,** 26.1 mg, 48.5 µmol), 2-(*tert*-butoxycarbonylamino)-2-methyl-propanoic acid (CAS: 30992-29-1, Sigma-Aldrich, Catalog: 15466, 11.8 mg, 58.2 µmol) and HATU in DMF (5 mL) was added DIEA (18.8 mg, 145.5 µmol). The reaction mixture was stirred at room temperature for 2 hrs, then quenched with water, extracted with EA. The organic layer was washed with brine, dried over Na₂SO₄, and then concentrated in *vacuo* to afford **compound 13.3** (29.7 mg, 80% yield). MS: calc'd 624 [(M+H)⁺], measured 624 [(M+H)⁺].

### Step (d): preparation of 5-[(4R,11aS)-9-[4-(2-amino-2-methyl-propanoyl)piperazin-1-yl]-4-methyl-1,3,4,6,11,11a-hexahydropyrazino[1,2-b]isoquinolin-2-yl]quinoline-8-carbonitrile (Example 13)

To a solution of *tert*-butyl *N*-[1,1-dimethyl-2-oxo-2-[4-[(4*R*,11a*S*)-2-(8-cyano-5-quinolyl)-4-methyl-1,3,4,6,11,11a-hexahydropyrazino[1,2-b]isoquinolin-9-yl]piperazin-1-yl]ethyl]carbamate **(compound 13.3,** 29.7 mg, 47.7 µmol ) in DCM (2 mL) was added TFA (1 mL). The reaction mixture was stirred at room temperature for 30 min, then concentrated to afford a crude product, which was purified by prep-HPLC to afford **Example 13** (11.9 mg, 48% yield). MS: calc'd 524 [(M+H)⁺], measured 524 [(M+H)⁺]. ¹H NMR (METHANOL-d₄, 400 MHz) δ 9.00 (dd, 1H, *J*=1.6, 4.3 Hz), 8.68 (dd, 1H, *J*=1.7, 8.6 Hz), 8.18 (d, 1H, *J*=8.1 Hz), 7.67 (dd, 1H, *J*=4.2, 8.6 Hz), 7.30 (d, 1H, *J*=8.1 Hz), 7.08 (d, 1H, *J*=8.4 Hz), 6.88 (dd, 1H, *J*=2.3, 8.3 Hz), 6.77 (d, 1H, *J*=2.1 Hz), 4.35 (d, 1H, *J*=14.8 Hz), 3.92 (br s, 4H), 3.6-3.7 (m, 1H), 3.49 (dd, 1H, *J*=2.1, 9.1 Hz), 3.40 (d, 1H, *J*=14.7 Hz), 3.1-3.2 (m, 4H), 3.0-3.1 (m, 1H), 2.8-3.0 (m, 4H), 2.7-2.8 (m, 1H), 1.47 (s, 6H), 1.32 (d, 3H, *J*=5.6 Hz)

### Example 14

### 5-[(4R,11aS)-4-methyl-9-[4-[(2S)-2-methylpyrrolidine-2-carbonyl]piperazin-1-yl]-1,3,4,6,11,11a-hexahydropyrazino[1,2-b]isoquinolin-2-yl]quinoline-8-carbonitrile

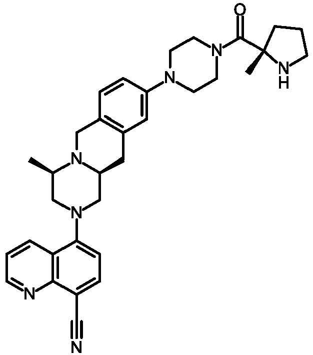

The title compound was prepared in analogy to the preparation of **Example 13** by using (2*S*)-1-*tert*-butoxycarbonyl-2-methyl-pyrrolidine-2-carboxylic acid (CAS: 103336-06-7, PharmaBlock, Catalog: PBN20121551) instead of 2-(*tert*-butoxycarbonylamino)-2-methyl-propanoic acid in step (c). **Example 14** was obtained. MS: calc'd 550 [(M+H)⁺], measured 550 [(M+H)⁺]. ¹H NMR (METHANOL-d₄, 400 MHz) δ 9.05 (dd, 1H, *J*=1.6, 4.3 Hz), 8.73 (dd, 1H, *J*=1.7, 8.6 Hz), 8.24 (d, 1H, *J*=7.9 Hz), 7.72 (dd, 1H, *J*=4.2, 8.6 Hz), 7.42 (d, 1H, *J*=8.1 Hz), 7.24 (d, 1H, *J*=8.6 Hz), 7.03 (dd, 1H, *J*=2.3, 8.6 Hz), 6.89 (d, 1H, *J*=2.2 Hz), 4.96 (d, 1H, *J*=15.2 Hz), 4.39 (br d, 1H, *J*=15.2 Hz), 4.1-4.3 (m, 1H), 3.9-4.0 (m, 2H), 3.7-3.9 (m, 5H), 3.4-3.5 (m, 2H), 3.2-3.3 (m, 7H), 3.0-3.2 (m, 1H), 2.3-2.5 (m, 2H), 2.0-2.3 (m, 2H), 1.76 (s, 3H), 1.6-1.7 (m, 3H).

### Example 15

### 5-[(4R,11aS)-9-[(1S,4S)-5-(2-amino-2-methyl-propanoyl)-2,5-diazabicyclo[2.2.1]heptan-2-yl]-4-methyl-1,3,4,6,11,11a-hexahydropyrazino[1,2-b]isoquinolin-2-yl]quinoline-8-carbonitrile

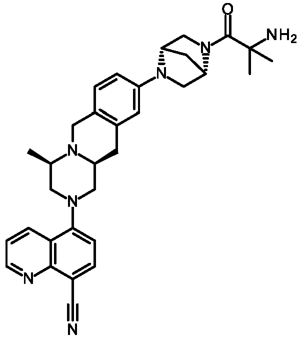

The title compound was prepared in analogy to the preparation of **Example 13** by using *tert*-butyl (1*S*,4*S*)-2,5-diazabicyclo[2.2.1]heptane-2-carboxylate (CAS: 113451-59-5, PharmaBlock, Catalog: PBN20120579) instead of *tert-*butyl piperazine-1-carboxylate in step (a). **Example 15** was obtained. MS: calc'd 536 [(M+H)⁺], measured 536 [(M+H)⁺]. ¹H NMR (METHANOL-d₄, 400 MHz) δ 9.07 (dd, 1H, *J*=1.6, 4.3 Hz), 8.78 (br d, 1H, *J*=8.6 Hz), 8.26 (d, 1H, *J*=7.8 Hz), 7.6-7.9 (m, 1H), 7.44 (d, 1H, *J*=7.7 Hz), 7.18 (d, 1H, *J*=8.7 Hz), 6.69 (dd, 1H, *J*=2.3, 8.6 Hz), 6.55 (br s, 1H), 5.03 (s, 1H), 4.90 (br, d, 1H, *J*=14.9 Hz), 4.6-4.7 (m, 1H), 4.36 (d, 1H, *J*=14.9 Hz), 4.1-4.2 (m, 1H), 3.9-4.1 (m, 2H), 3.4-3.8 (m, 4H), 3.2-3.3 (m, 3H), 3.1-3.2 (m, 2H), 2.0-2.2 (m, 2H), 1.71 (br s, 6H), 1.63 (d, 3H, *J*=6.5 Hz).

### Example 16

### 5-[(4R,11aS)-9-(4-amino-2-oxo-pyrrolidin-1-yl)-4-methyl-1,3,4,6,11,11a-hexahydropyrazino[1,2-b]isoquinolin-2-yl]quinoline-8-carbonitrile

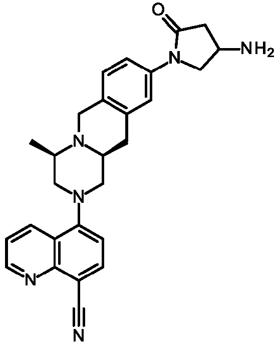

The titled compound was synthesized according to the following scheme:

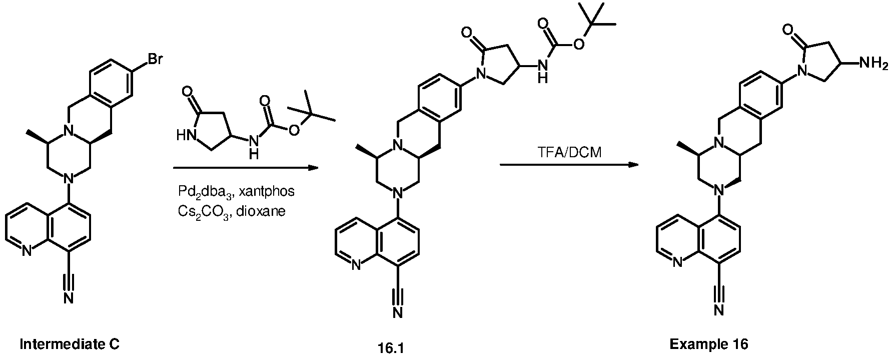

### Step (a): preparation of tert-butyl N-[5-oxo-1-[(4R,11aS)-2-(8-cyano-5-quinolyl)-4-methyl-1,3,4,6,11,11a-hexahydropyrazino[1,2-b]isoquinolin-9-yl]pyrrolidin-3-yl]carbamate (compound 16.1)

To a solution of *tert*-butyl *N*-(5-oxopyrrolidin-3-yl)carbamate (CAS: 1245648-84-3, BePharm, Catalog: BD165019, 60 mg, 299 µmol) in dioxane (5 mL) was added 5-((4R,11aS)-8-bromo-4-methyl-1,3,4,6,11,11a-hexahydro-2*H*-pyrazino[1,2-b]isoquinolin-2-yl)quinoline-8-carbonitrile **(Intermediate C,** 129.6 mg, 299 µmol), Cs₂CO₃ (136.4 mg, 418.6 µmol) and Pd₂(dba)₃ (13.7 mg, 14.9 µmol) and Xantphos (CAS: 51364-51-3, Sigma-Aldrich, Catalog: 526460, 25.9 mg, 44.7 µmol). The resultant mixture was stirred at 120 °C overnight. After being cooled to room temperature, the mixture was diluted with water (20 mL), and extracted with EA (20 mL) for three times. The combined organic layer was washed with brine, dried over Na₂SO₄, filtered and concentrated *in vacuo.* The residue was purified by flash chromatography (silica gel, 24 g, 0% to 100% EtOAc in PE) to afford **compound 16.1** (76.5 mg, 79 % yield). MS: calc'd 553 [(M+H)⁺], measured 553 [(M+H)⁺].

### Step (b): preparation of 5-[(4R,11aS)-9-(4-amino-2-oxo-pyrrolidin-1-yl)-4-methyl-1,3,4,6,11,11a-hexahydropyrazino[1,2-b]isoquinolin-2-yl]quinoline-8-carbonitrile (Example 16)

To a solution of *tert-butyl* N-[5-oxo-1-[(4*R*,11a*S*)-2-(8-cyano-5-quinolyl)-4-methyl-1,3,4,6,11,11a-hexahydropyrazino[1,2-b]isoquinolin-9-yl]pyrrolidin-3-yl]carbamate **(compound 16.1,** 76.5 mg, 138.4 µmol ) in DCM (2 mL) was added TFA (1 mL). The reaction mixture was stirred at room temperature for 30 min, then concentrated to afford a crude product, which was purified by prep-HPLC to afford **Example 16** ( 28.3 mg, 45.1% yield). MS: calc'd 453 [(M+H)⁺], measured 453 [(M+H)⁺]. ¹H NMR (METHANOL-d₄, 400 MHz) δ 9.06 (dd, 1H, *J*=1.5, 4.3 Hz), 8.73 (dd, 1H, *J*=1.6, 8.7 Hz), 8.25 (d, 1H, *J*=7.9 Hz), 7.5-7.9 (m, 3H), 7.3-7.5 (m, 2H), 5.07 (br d, 1H, J=15.4 Hz), 4.3-4.6 (m, 2H), 4.1-4.3 (m, 2H), 3.9-4.1 (m, 3H), 3.82 (br d, 1H, J=12.7 Hz), 3.1-3.3 (m, 5H), 2.69 (dd, 1H, J=3.3, 18.0 Hz), 1.64 (d, 3H, J=6.5 Hz)

### Example 17A and 17B

### 5-[(4R,11aS)-4-methyl-9-[(8aS)-3-oxo-1,5,6,7,8,8a-hexahydroimidazo[1,5-a]pyrazin-2-yl]-1,3,4,6,11,11a-hexahydropyrazino[1,2-b]isoquinolin-2-yl]quinoline-8-carbonitrile and 5-[(4R,11aS)-4-methyl-9-[(8aR)-3-oxo-1,5,6,7,8,8a-hexahydroimidazo[1,5-a]pyrazin-2-yl]-1,3,4,6,11,11a-hexahydropyrazino[1,2-b]isoquinolin-2-yl]quinoline-8-carbonitrile

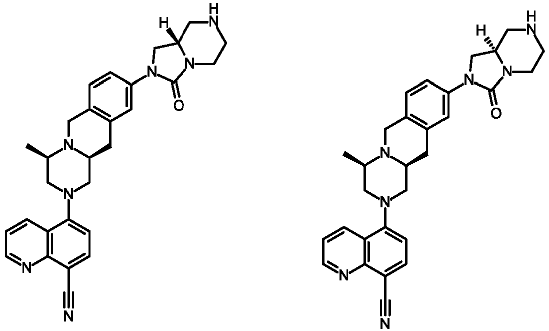

The title compounds were prepared according to the following scheme:

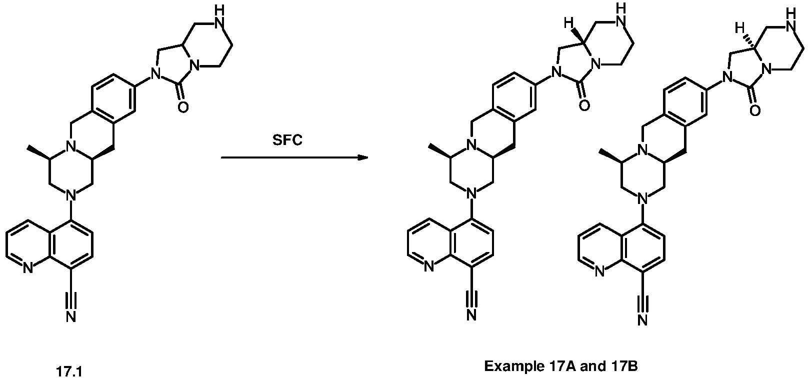

The **compound 17.1** was prepared in analogy to the preparation of **Example 16** by using *tert-butyl* 3-oxo-1,2,5,6,8,8a-hexahydroimidazo[1,5-a]pyrazine-7-carboxylate (CAS: 1246551-25-6, BePharm, Catalog: BD286163) instead of *tert*-butyl *N*-(5-oxopyrrolidin-3-yl)carbamate in step (a). Then **Compound 17.1** was resolved by SFC to give two single isomers: **Example 17A** (faster eluting) and **Example 17B** (slower eluting) with 30% methanol (0.1% NH₃H₂O)/CO₂ on DAICEL CHIRALPAK AD (10 µm, 250×30 mm) column.

**Example 17A** MS: calc'd 494 [(M+H)⁺], measured 494 [(M+H)⁺]. ¹H NMR (METHANOL-d₄, 400 MHz) δ 8.92 (dd, 1H, *J*=1.2, 4.2 Hz), 8.61 (dd, 1H, *J*=1.3, 8.6 Hz), 8.11 (d, 1H, *J*=8.1 Hz), 7.60 (dd, 1H, *J*=4.3, 8.6 Hz), 7.44 (s, 2H), 7.30 (d, 1H, *J*=7.9 Hz), 7.21 (d, 1H, *J*=9.0 Hz), 4.90 (br d, 1H, *J*=15.4 Hz), 4.33 (br d, 1H, *J*=15.4 Hz), 3.6-4.2 (m, 8H), 3.5-3.6 (m, 1H), 3.44 (br d, 1H, *J*=12.3 Hz), 3.31 (br d, 1H, *J*=12.6 Hz), 3.23 (br d, 1H, *J*=3.8 Hz), 3.1-3.2 (m, 3H), 2.9-3.1 (m, 3H), 1.52 (d, 3H, *J*=6.5 Hz).

**Example 17B** MS: calc'd 494 [(M+H)⁺], measured 494 [(M+H)⁺]. ¹H NMR (METHANOL-d₄, 400 MHz) δ 9.06 (dd, 1H, *J*=1.5, 4.2 Hz), 8.73 (dd, 1H, *J*=1.7, 8.6 Hz), 8.25 (d, 1H, *J*=7.9 Hz), 7.73 (dd, 1H, *J*=4.3, 8.6 Hz), 7.5-7.6 (m, 2H), 7.43 (d, 1H, *J*=7.9 Hz), 7.34 (d, 1H, *J*=8.9 Hz), 5.02 (br d, 1H, *J*=15.8 Hz), 4.44 (br d, 1H, *J*=15.4 Hz), 4.1-4.3 (m, 6H), 3.9-4.1 (m, 2H), 3.7-3.9 (m, 2H), 3.4-3.6 (m, 2H), 3.0-3.3 (m, 6H), 1.63 (d, 3H, *J*=6.4 Hz).

### Example 18

### 5-[(4R,11aS)-4-methyl-9-(3-oxo-3a,4,5,6,7,7a-hexahydro-1H-pyrrolo[3,4-c]pyridin-2-yl)-1,3,4,6,11,11a-hexahydropyrazino[1,2-b]isoquinolin-2-yl]quinoline-8-carbonitrile

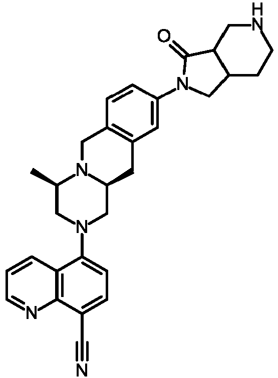

The title compound was prepared in analogy to the preparation of **Example 16** by using *tert*-butyl 3-oxo-2,3a,4,6,7,7a-hexahydro-1*H*-pyrrolo[3,4-c]pyridine-5-carboxylate (CAS: 1780976-05-7, PharmaBlock, Catalog: PBWBD0104) instead of *tert*-butyl *N*-(5-oxopyrrolidin-3-yl)carbamate in in step (a). **Example 18** was obtained. MS: calc'd 493 [(M+H)⁺], measured 493 [(M+H)⁺]. ¹H NMR (METHANOL-d₄, 400 MHz) δ 9.06 (d, 1H, *J*=4.2 Hz), 8.73 (dd, 1H, *J*=1.6, 8.6 Hz), 8.25 (d, 1H, *J*=8.1 Hz), 7.6-7.8 (m, 3H), 7.41 (dd, 2H, *J*=8.2, 15.8 Hz), 5.06 (br d, 1H, *J*=15.9 Hz), 4.47 (br d, 1H, *J*=15.7 Hz), 4.2-4.3 (m, 1H), 4.12 (td, 1H, *J*=4.9, 10.1 Hz), 3.9-4.1 (m, 2H), 3.82 (br d, 1H, *J*=13.3 Hz), 3.5-3.6 (m, 2H), 3.4-3.4 (m, 1H), 3.1-3.3 (m, 5H), 2.8-3.0 (m, 3H), 2.39 (br dd, 1H, *J*=1.8, 15.2 Hz), 2.0-2.2 (m, 1H), 1.64 (d, 3H, *J*=6.5 Hz).

### Example 19

### 5-[(4R,11aS)-4-methyl-9-(1-oxo-3a,4,5,6,7,7a-hexahydro-3H-pyrrolo[3,4-c]pyridin-2-yl)-1,3,4,6,11,11a-hexahydropyrazino[1,2-b]isoquinolin-2-yl]quinoline-8-carbonitrile

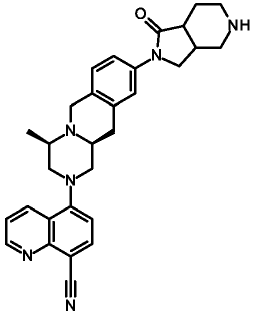

The title compound was prepared in analogy to the preparation of **Example 16** by using *tert*-butyl 1-oxo-3,3a,4,6,7,7a-hexahydro-2*H*-pyrrolo[3,4-c]pyridine-5-carboxylate (CAS: 1781135-05-4, PharmaBlock, Catalog: PBWBD0105) instead of *tert-butyl* N-(5-oxopyrrolidin-3-yl)carbamate in in step (a). **Example 19** was obtained. MS: calc'd 493 [(M+H)⁺], measured 493 [(M+H)⁺]. ¹H NMR (METHANOL-d₄, 400 MHz) δ 9.06 (d, 1H, *J*=4.0 Hz), 8.73 (d, 1H, *J*=8.6 Hz), 8.25 (d, 1H, *J*=7.8 Hz), 7.6-7.8 (m, 3H), 7.41 (dd, 2H, *J*=8.4, 15.8 Hz), 5.05 (br d, 1H, *J*=15.3 Hz), 4.46 (br d, 1H, *J*=15.5 Hz), 4.1-4.3 (m, 2H), 3.9-4.1 (m, 2H), 3.82 (br d, 2H, *J*=13.1 Hz), 3.58 (br dd, 2H, *J*=4.0, 10.2 Hz), 3.0-3.3 (m, 8H), 2.7-2.9 (m, 1H), 2.1-2.2 (m, 1H), 1.64 (d, 3H, *J*=6.5 Hz).

### Example 20

The following tests were carried out in order to determine the activity of the compounds of formula (I), (Ia) or (Ib) in HEK293-Blue-hTLR-7/8/9 cells assay.

### HEK293-Blue-hTLR-7 cells assay:

A stable HEK293-Blue-hTLR-7 cell line was purchased from InvivoGen (Cat.#: hkb-htlr7, San Diego, California, USA). These cells were originally designed for studying the stimulation of human TLR7 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR7 cells with TLR7 ligands. Therefore the reporter expression was declined by TLR7 antagonist under the stimulation of a ligand, such as R848 (Resiquimod), for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR7 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM R848 in above DMEM, perform incubation under 37 °C in a CO₂ incubator for 20 hrs. Then 20 µL of the supernatant from each well was incubated with 180 µL Quanti-blue substrate solution at 37 °C for 2 hrs and the absorbance was read at 620~655 nm using a spectrophotometer. The signaling pathway that TLR7 activation leads to downstream NF-κB activation has been widely accepted, and therefore similar reporter assay was modified for evaluating TLR7 antagonist.

### HEK293-Blue-hTLR-8 cells assay:

A stable HEK293-Blue-hTLR-8 cell line was purchased from InvivoGen (Cat.#: hkb-htlr8, San Diego, California, USA). These cells were originally designed for studying the stimulation of human TLR8 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-xB and AP-*1 via* stimulating HEK-Blue hTLR8 cells with TLR8 ligands. Therefore the reporter expression was declined by TLR8 antagonist under the stimulation of a ligand, such as R848, for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR8 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 60uM R848 in above DMEM, perform incubation under 37 °C in a CO₂ incubator for 20 hrs. Then 20 µL of the supernatant from each well was incubated with 180 µL Quanti-blue substrate solution at 37°C for 2 hrs and the absorbance was read at 620~655 nm using a spectrophotometer. The signaling pathway that TLR8 activation leads to downstream NF-κB activation has been widely accepted, and therefore similar reporter assay was modified for evaluating TLR8 antagonist.

### HEK293-Blue-hTLR-9 cells assay:

A stable HEK293-Blue-hTLR-9 cell line was purchased from InvivoGen (Cat.#: hkb-htlr9, San Diego, California, USA). These cells were originally designed for studying the stimulation of human TLR9 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-*1 via* stimulating HEK-Blue hTLR9 cells with TLR9 ligands. Therefore the reporter expression was declined by TLR9 antagonist under the stimulation of a ligand, such as ODN2006 (Cat.#: tlrl-2006-1, Invivogen, San Diego, California, USA), for incubation of 20 hrs. The cell culture supernatant SEAP reporter activity was determined using QUANTI-Blue^{™} kit (Cat.#: rep-qb1, Invivogen, San Diego, California, USA) at a wavelength of 640 nm, a detection medium that turns purple or blue in the presence of alkaline phosphatase.

HEK293-Blue-hTLR9 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM ODN2006 in above DMEM, perform incubation under 37 °C in a CO₂ incubator for 20 hrs. Then 20 µL of the supernatant from each well was incubated with 180 µL Quanti-blue substrate solution at 37 °C for 2 h and the absorbance was read at 620~655 nm using a spectrophotometer. The signaling pathway that TLR9 activation leads to downstream NF-κB activation has been widely accepted, and therefore similar reporter assay was modified for evaluating TLR9 antagonist.

The compounds of formula (I) have TLR7 and/or TLR8 inhibitory activities (IC₅₀ value) <0.1 µM. Moreover, most compounds also have TLR9 inhibitory activity <0.3 µM. Activity data of the compounds of the present invention were shown in Table 1.

**Table 1: The activity of the compounds of present invention in HEK293-Blue-hTLR-7/8/9 cells assays**

| Example No | HEK/hTLR7 IC₅₀ (nM) | HEK/hTLR8 IC₅₀ (nM) | HEK/hTLR9 IC₅₀ (nM) |
|---|---|---|---|
| 1 | 71.6 | 14.5 | 64.6 |
| 2 | 53.1 | 4.2 | 44.5 |
| 3A | 33.2 | 4.5 | 77.3 |
| 3B | 60.7 | 5.7 | 103.1 |
| 4A | 23.2 | <3.2 | 97.5 |
| 4B | 31.8 | 7.8 | 85.5 |
| 5 | 38.2 | 9.2 | 96.3 |
| 6 | 24.7 | 26.1 | 106.4 |
| 7 | 27.4 | 13.5 | 109.0 |
| 8 | 35.5 | 21.5 | 85.3 |
| 9 | 19.5 | 2.3 | 138.9 |
| 10 | 21.8 | 1.5 | 78.2 |
| 11 | 5.8 | 11.1 | 81.0 |
| 12 | 18.2 | 22.9 | 116.5 |
| 13 | 18.6 | 37.5 | 85.6 |
| 14 | 18.6 | 1.4 | 254.1 |
| 15 | 2.4 | 4.7 | 125.2 |
| 16 | 2.9 | 5.3 | 125.4 |
| 17A | 10.3 | 8.0 | 106.7 |
| 17B | 10.3 | 2.4 | 106.4 |
| 18 | 71.6 | 14.5 | 64.6 |
| 19 | 53.1 | 4.2 | 44.5 |

### Example 21

### Human Microsomal Stability Assay

Human liver microsomes (Cat.NO.: 452117, Corning, USA) were preincubated with test compound for 10 minutes at 37°C in 100 mM potassium phosphate buffer, pH 7.4. The reactions were initiated by adding NADPH regenerating system. The final incubation mixtures contained 1 µM test compound, 0.5 mg/mL liver microsomal protein, 1 mM MgCl₂, 1 mM NADP, 1 unit/mL isocitric dehydrogenase and 6 mM isocitric acid in 100 mM potassium phosphate buffer, pH 7.4. After incubation times of 0, 3, 6, 9, 15 and 30 minutes at 37°C, 300 µL of cold ACN (including internal standard) was added to 100 µL incubation mixture to terminate the reaction. Following precipitation and centrifugation, 100uL supernatant will be taken out and added 300uL water. The amount of compound remaining in the samples was determined by LC-MS/MS. Controls of no NADPH regenerating system at zero and 30 minutes were also prepared and analyzed. The results were categorized as: low (<7.0 mL/min/kg), medium (7.0-16.2 mL/min/kg) and high (16.2-23.2 mL/min/kg). Test results were summarized in Table 2.

**Table 2: Human microsomal stability results**

| Example No | CL (h) (mL/min/kg) |
|---|---|
| 6 | 6.8 |
| 8 | 9.6 |
| 9 | 8.8 |
| 10 | 9.3 |
| 13 | 7.6 |
| 14 | 8.7 |
| 16 | 6.8 |

### Example 22

### hERG Channel Inhibition Assay

The hERG channel inhibition assay is a highly sensitive measurement that identifies compounds exhibiting hERG inhibition related to cardiotoxicity in vivo. The hERG K⁺ channels were cloned in humans and stably expressed in a CHO ( Chinese hamster ovary) cell line. CHO_{hERG} cells were used for patch-clamp (voltage-clamp, whole-cell) experiments. Cells were stimulated by a voltage pattern to activate hERG channels and conduct I_{KhERG} currents (rapid delayed outward rectifier potassium current of the hERG channel). After the cells were stabilized for a few minutes, the amplitude and kinetics of I_{KhERG} were recorded at a stimulation frequency of 0.1 Hz (6 bpm). Thereafter, the test compound was added to the preparation at increasing concentrations. For each concentration, an attempt was made to reach a steady-state effect, usually, this was achieved within 3-10 min at which time the next highest concentration was applied. The amplitude and kinetics of I_{KhERG} are recorded in each concentration of the drug which were compared to the control values (taken as 100%). (references: Redfern WS, Carlsson L, Davis AS, Lynch WG, MacKenzie I, Palethorpe S, Siegl PK, Strang I, Sullivan AT, Wallis R, Camm AJ, Hammond TG. 2003; Relationships between preclinical cardiac electrophysiology, clinical QT interval prolongation and torsade de pointes for a broad range of drugs: evidence for a provisional safety margin in drug development. Cardiovasc. Res. 58:32-45, Sanguinetti MC, Tristani-Firouzi M. 2006; hERG potassium channels and cardiac arrhythmia. Nature 440:463-469, Webster R, Leishman D, Walker D. 2002; Towards a drug concentration effect relationship for QT prolongation and torsades de pointes. Curr. Opin. Drug Discov. Devel. 5:116-26). Results of hERG are given in Table 3.

**Table 3: hERG results**

| Example No | hERG IC₂₀ (µM) | hERG IC₅₀ (µM) |
|---|---|---|
| 6 | >10 | >20 |
| 12 | 8.2 | >20 |
| 17A | >10 | >20 |

## Claims

1. A compound of formula (I), 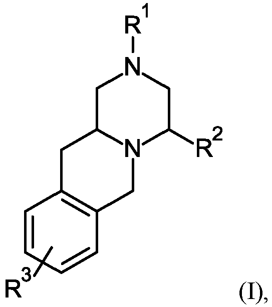 wherein
R¹ is 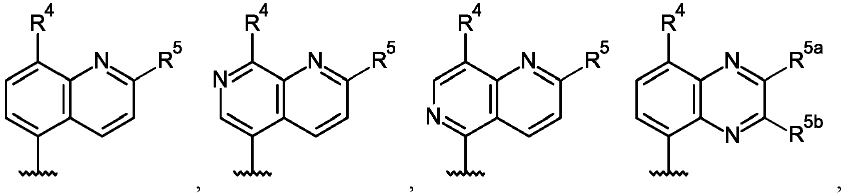 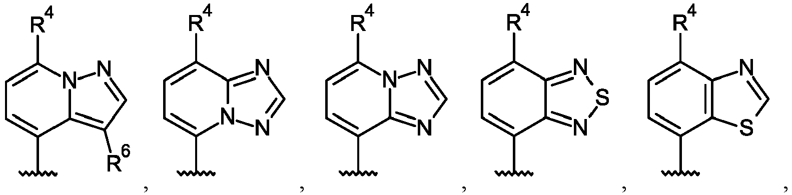 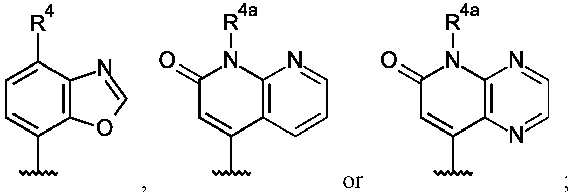 wherein R⁴ is C₁₋₆alkyl, C₁₋₆alkoxy, haloC₁₋₆alkyl, halogen, nitro or cyano; R^{4a} is C₁₋₆alkyl or C₃₋₇cycloalkyl; R⁵, R^{5a} and R^{5b} are independently selected from H and deuterium; R⁶ is H or halogen;
R² is C₁₋₆alkyl;
R³ is unsubstituted or substituted heterocyclyl, heterocyclylamino, heterocyclylC₁₋₆alkylamino or substituted C₁₋₆alkoxy;
or a pharmaceutically acceptable salt thereof.

2. A compound according to claim 1, wherein R¹ is 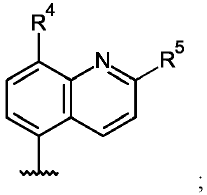 wherein R⁴ is cyano, R⁵ is H or deuterium.

3. A compound according to claim 2, wherein R³ is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; (hydroxypyrrolidinyl)C₁₋₆alkylamino; (morpholinyl)C₁₋₆alkylamino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; amino-1,4-oxazepanyl; aminohalopyrrolidinyl; aminooxopyrrolidinyl or azetidinylC₁₋₆alkoxy.

4. A compound according to claim 1, wherein the compound is of formula (Ia), 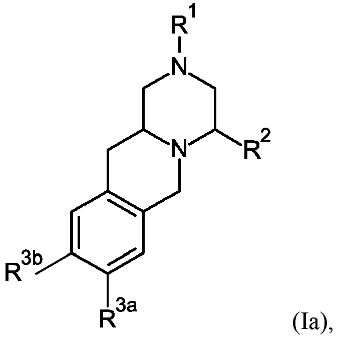 wherein
R¹ is 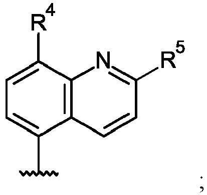 wherein R⁴ is cyano, R⁵ is H or deuterium;
R² is C₁₋₆alkyl;
a) R^{3a} is (C₁₋₆alkyl)₂aminoC₁₋₆alkoxy; (halopyrrolidinyl)amino; (hydroxypyrrolidinyl)C₁₋₆alkylamino; (morpholinyl)C₁₋₆alkylamino or amino-1,4-oxazepanyl;
R^{3b} is H; or
b) R^{3a} is H;
R^{3b} is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; aminohalopyrrolidinyl; aminooxopyrrolidinyl or azetidinylC₁₋₆alkoxy;
or a pharmaceutically acceptable salt thereof.

5. A compound according to claim 1, wherein the compound is of formula (Ib), 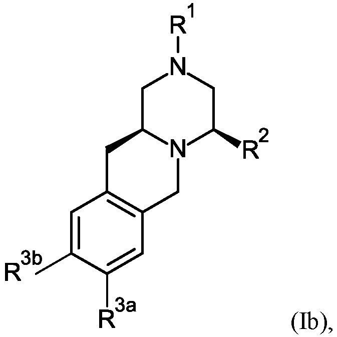 wherein
R¹ is 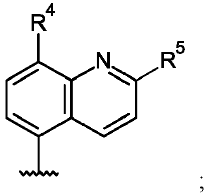 wherein R⁴ is cyano, R⁵ is H or deuterium;
R² is C₁₋₆alkyl;
a) R^{3a} is (C₁₋₆alkyl)₂aminoC₁₋₆alkoxy; (halopyrrolidinyl)amino; (hydroxypyrrolidinyl)C₁₋₆alkylamino; (morpholinyl)C₁₋₆alkylamino or amino-1,4-oxazepanyl;
R^{3b} is H; or
b) R^{3a} is H;
R^{3b} is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; aminohalopyrrolidinyl; aminooxopyrrolidinyl or azetidinylC₁₋₆alkoxy;
or a pharmaceutically acceptable salt thereof.

6. A compound according to claim 4 or 5, wherein
R¹ is 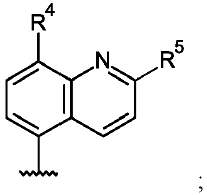 wherein R⁴ is cyano, R⁵ is H or deuterium;
R² is C₁₋₆alkyl;
R^{3a} is H;
R^{3b} is (aminoC₁₋₆alkylcarbonyl)-2,5-diazabicyclo[2.2.1]heptanyl; (aminoC₁₋₆alkylcarbonyl)piperazinyl; (C₁₋₆alkoxypyrrolidinyl)amino; (C₁₋₆alkyl)₂aminoC₁₋₆alkoxy; (C₁₋₆alkylpyrrolidinylcarbonyl)piperazinyl; (halopyrrolidinyl)amino; 1-oxo-3a,4,5,6,7,7a-hexahydro-3H-pyrrolo[3,4-c]pyridinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl; 3-oxo-3a,4,5,6,7,7a-hexahydro-1H-pyrrolo[3,4-c]pyridinyl; amino(C₁₋₆alkoxy)pyrrolidinyl; aminohalopyrrolidinyl; aminooxopyrrolidinyl or azetidinylC₁₋₆alkoxy;
or a pharmaceutically acceptable salt thereof.

7. A compound according to claim 6, wherein R² is methyl.

8. A compound according to claim 6, wherein R^{3b} is (2-amino-2-methyl-propanoyl)-2,5-diazabicyclo[2.2.1]heptan-2-yl; (2-amino-2-methyl-propanoyl)piperazin-1-yl; (2-methylpyrrolidine-2-carbonyl)piperazin-1-yl; (4-fluoropyrrolidin-3-yl)amino; (4-methoxypyrrolidin-3-yl)amino; (azetidin-2-yl)methoxy; 1-oxo-3a,4,5,6,7,7a-hexahydro-3H-pyrrolo[3,4-c]pyridin-2-yl; 2-(dimethylamino)ethoxy; 3,4,4a,5,7,7a-hexahydro-2H-pyrrolo[3,4-b][1,4]oxazin-6-yl; 3-amino-4-fluoro-pyrrolidin-1-yl; 3-amino-4-methoxy-pyrrolidin-1-yl; 3-oxo-1,5,6,7,8,8a-hexahydroimidazo[1,5-a]pyrazin-2-yl; 3-oxo-3a,4,5,6,7,7a-hexahydro-1H-pyrrolo[3,4-c]pyridin-2-yl or 4-amino-2-oxo-pyrrolidin-1-yl.

9. A compound according to claim 6, wherein R^{3b} is (C₁₋₆alkylpyrrolidinylcarbonyl)piperazinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl or azetidinylC₁₋₆alkoxy.

10. A compound according to claim 9, wherein R^{3b} is (2-methylpyrrolidine-2-carbonyl)piperazin-1-yl; 3,4,4a,5,7,7a-hexahydro-2H-pyrrolo[3,4-b][1,4]oxazin-6-yl; 3-oxo-1,5,6,7,8,8a-hexahydroimidazo[1,5-a]pyrazin-2-yl or (azetidin-2-yl)methoxy.

11. A compound according to claim 4 or 5, wherein
R¹ is 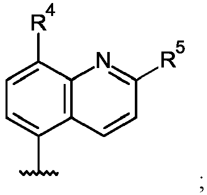 wherein R⁴ is cyano, R⁵ is H or deuterium;
R² is C₁₋₆alkyl;
R^{3a} is H;
R^{3b} is (C₁₋₆alkylpyrrolidinylcarbonyl)piperazinyl; 3,4,4a,5,7,7a-hexahydro-2H-pyrrolo[3,4-b][1,4]oxazinyl; 3-oxo-1,5,6,7,8,8a-hexahydroimidazo[1,5-a]pyrazinyl or azetidinylC₁₋₆alkoxy;
or a pharmaceutically acceptable salt thereof.

12. A compound according to claim 11, wherein
R¹ is 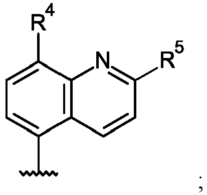 wherein R⁴ is cyano, R⁵ is H or deuterium;
R² is methyl;
R^{3a} is H;
R^{3b} is (2-methylpyrrolidine-2-carbonyl)piperazin-1-yl; 3,4,4a,5,7,7a-hexahydro-2H-pyrrolo[3,4-b][1,4]oxazin-6-yl; 3-oxo-1,5,6,7,8,8a-hexahydroimidazo[1,5-a]pyrazin-2-yl or (azetidin-2-yl)methoxy;
or a pharmaceutically acceptable salt thereof.

13. A compound according to any one of claims 1 to 12, wherein the compound is selected from:
5-[(4*R*,11a*S*)-8-(6-amino-1,4-oxazepan-4-yl)-4-methyl-1,3,4,6,11,11a-hexahydropyrazino[1,2-b]isoquinolin-2-yl]quinoline-8-carbonitrile;
5-[(4*R*,11a*S*)-8-[2-[(3*S*)-3-hydroxypyrrolidin-1-yl]ethylamino]-4-methyl-1,3,4,6,11,11a-hexahydropyrazino[1,2-b]isoquinolin-2-yl]quinoline-8-carbonitrile;
5-[(4*R*,11a*S*)-4-methyl-8-[[(2*R*)-morpholin-2-yl]methylamino]-1,3,4,6,11,11a-hexahydropyrazino[1,2-b]isoquinolin-2-yl]quinoline-8-carbonitrile;
5-[(4*R*,11a*S*)-8-[[(3*S*,4*R*)-4-fluoropyrrolidin-3-yl]amino]-4-methyl-1,3,4,6,11,11a-hexahydropyrazino[1,2-b]isoquinolin-2-yl]quinoline-8-carbonitrile;
5-[(4*R*,11a*S*)-8-[2-(dimethylamino)ethoxy]-4-methyl-1,3,4,6,11,11a-hexahydropyrazino[1,2-b]isoquinolin-2-yl]quinoline-8-carbonitrile;
5-[(4*R*,11a*S*)-4-methyl-9-[[(3*R*,4*S*)-4-fluoropyrrolidin-3-yl]amino]-1,3,4,6,11,11a-hexahydropyrazino[1,2-b]isoquinolin-2-yl]quinoline-8-carbonitrile;
5-[(4*R*,11a*S*)-9-[[(3*R*,4*S*)-4-methoxypyrrolidin-3-yl]amino]-4-methyl-1,3,4,6,11,11a-hexahydropyrazino[1,2-b]isoquinolin-2-yl]quinoline-8-carbonitrile;
5-[(4*R*,11a*S*)-9-[(3*S*,4*R*)-3-amino-4-fluoro-pyrrolidin-1-yl]-4-methyl-1,3,4,6,11,11a-hexahydropyrazino[1,2-b]isoquinolin-2-yl]quinoline-8-carbonitrile;
5-[(4*R*,11a*S*)-9-[(3*R*,4*S*)-3-amino-4-methoxy-pyrrolidin-1-yl]-4-methyl-1,3,4,6,11,11a-hexahydropyrazino[1,2-b]isoquinolin-2-yl]quinoline-8-carbonitrile;
5-[(4*R*,11a*S*)-9-[(4a*S*,7a*S*)-3,4,4a,5,7,7a-hexahydro-2*H*-pyrrolo[3,4-b][1,4]oxazin-6-yl]-4-methyl-1,3,4,6,11,11a-hexahydropyrazino[1,2-b]isoquinolin-2-yl]quinoline-8-carbonitrile;
5-[(4*R*,11a*S*)-9-[2-(dimethylamino)ethoxy]-4-methyl-1,3,4,6,11,11a-hexahydropyrazino[1,2-b]isoquinolin-2-yl]quinoline-8-carbonitrile;
5-[(4*R*,11a*S*)-9-[[(2*S*)-azetidin-2-yl]methoxy]-4-methyl-1,3,4,6,11,11a-hexahydropyrazino[1,2-b]isoquinolin-2-yl]quinoline-8-carbonitrile;
5-[(4*R*,11a*S*)-4-methyl-9-[[(3*R*,4*S*)-4-fluoropyrrolidin-3-yl]amino]-1,3,4,6,11,11a-hexahydropyrazino[1,2-b]isoquinolin-2-yl]quinoline-8-carbonitrile;
5-[(4*R*,11a*S*)-4-methyl-9-[4-[(2*S*)-2-methylpyrrolidine-2-carbonyl]piperazin-1-yl]-1,3,4,6,11,11a-hexahydropyrazino[1,2-b]isoquinolin-2-yl]quinoline-8-carbonitrile;
5-[(4*R*,11a*S*)-9-[(1*S*,4*S*)-5-(2-amino-2-methyl-propanoyl)-2,5-diazabicyclo[2.2.1]heptan-2-yl]-4-methyl-1,3,4,6,11,11a-hexahydropyrazino[1,2-b]isoquinolin-2-yl]quinoline-8-carbonitrile;
5-[(4*R*,11a*S*)-9-(4-amino-2-oxo-pyrrolidin-1-yl)-4-methyl-1,3,4,6,11,11a-hexahydropyrazino[1,2-b]isoquinolin-2-yl]quinoline-8-carbonitrile;
5-[(4*R*,11a*S*)-4-methyl-9-[(8a*S*)-3-oxo-1,5,6,7,8,8a-hexahydroimidazo[1,5-a]pyrazin-2-yl]-1,3,4,6,11,11a-hexahydropyrazino[1,2-b]isoquinolin-2-yl]quinoline-8-carbonitrile;
5-[(4*R*,11a*S*)-4-methyl-9-[(8a*R*)-3-oxo-1,5,6,7,8,8a-hexahydroimidazo[1,5-a]pyrazin-2-yl]-1,3,4,6,11,11a-hexahydropyrazino[1,2-b]isoquinolin-2-yl]quinoline-8-carbonitrile;
5-[(4*R*,11a*S*)-4-methyl-9-(3-oxo-3a,4,5,6,7,7a-hexahydro-1*H*-pyrrolo[3,4-c]pyridin-2-yl)-1,3,4,6,11,11a-hexahydropyrazino[1,2-b]isoquinolin-2-yl]quinoline-8-carbonitrile; and
5-[(4*R*,11a*S*)-4-methyl-9-(1-oxo-3a,4,5,6,7,7a-hexahydro-3*H*-pyrrolo[3,4-c]pyridin-2-yl)-1,3,4,6,11,11a-hexahydropyrazino[1,2-b]isoquinolin-2-yl]quinoline-8-carbonitrile;
or a pharmaceutically acceptable salt, enantiomer or diastereomer thereof.

14. A process for the preparation of a compound according to any one of claims 1 to 13 comprising the following step:
a) the reaction of compound of formula (V), 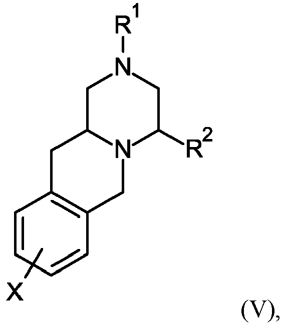 and R³-H via Buchwald-Hartwig amination or Suzuki coupling or Stille coupling or Negishi coupling; wherein R³-H is an amine or an alcohol;
wherein X is halogen or leaving group; R¹ to R³ are defined as in any one of claims 1 to 12.

15. A compound or pharmaceutically acceptable salt, enantiomer or diastereomer according to any one of claims 1 to 13 for use as therapeutically active substance.

16. A pharmaceutical composition comprising a compound according to any one of claims 1 to 13 and a therapeutically inert carrier.

17. A compound or pharmaceutically acceptable salt, enantiomer or diastereomer according to any one of claims 1 to 13 for use in the treatment or prophylaxis of systemic lupus erythematosus or lupus nephritis.

## Patentansprüche

1. Verbindung der Formel (I), 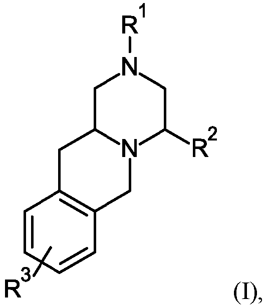 wobei
R¹ 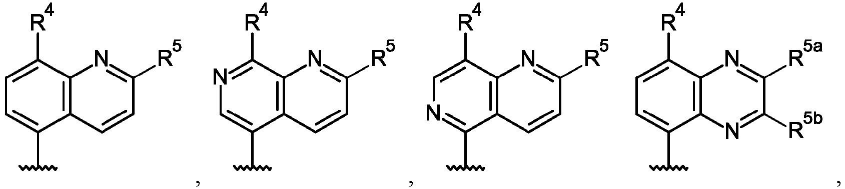 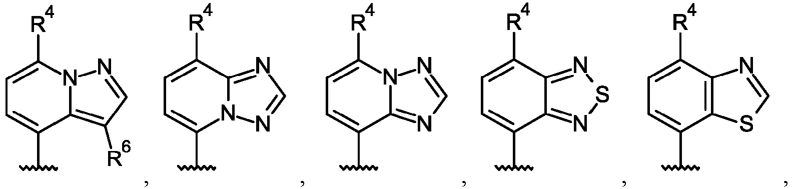 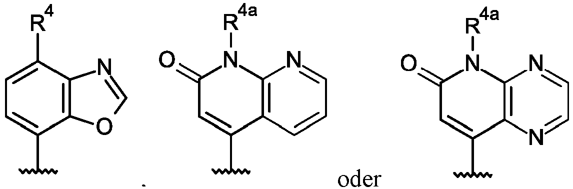 ist; wobei R⁴ C₁₋₆-Alkyl, C₁₋₆-Alkoxy, Halogen-C₁₋₆-alkyl, Halogen, Nitro oder Cyano ist; R^{4a} C₁₋₆-Alkyl oder C₃₋₇-Cycloalkyl ist; R⁵*,* R^{5a} und R^{5b} unabhängig voneinander aus H und Deuterium ausgewählt sind; R⁶ H oder Halogen ist;
R² C₁₋₆-Alkyl ist;
R³ unsubstituiertes oder substituiertes Heterocyclyl, Heterocyclylamino, Heterocyclyl-C₁₋₆-alkylamino oder substituiertes C₁₋₆-Alkoxy ist;
oder ein pharmazeutisch unbedenkliches Salz davon.

2. Verbindung nach Anspruch 1, wobei R¹ 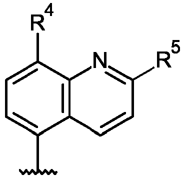 ist; wobei R⁴ Cyano ist, R⁵ H oder Deuterium ist.

3. Verbindung nach Anspruch 2, wobei R³ (Amino-C₁₋₆-alkylcarbonyl)-2,5-diazabicyclo[2.2.1]heptanyl; (Amino-C₁₋₆-alkylcarbonyl)piperazinyl, (C₁₋₆-Alkoxypyrrolidinyl)amino; (C₁₋₆₋Alkyl)₂-amino-C₁₋₆-alkoxy, (C₁₋₆-Alkylpyrrolidinylcarbonyl)piperazinyl; (Halogenpyrrolidinyl)amino; (Hydroxypyrrolidinyl)-C₁₋₆-alkylamino, (Morpholinyl)-C₁₋₆-alkylamino, 1-Oxo-3a,4,5,6,7,7a-hexahydro-3H-pyrrolo[3,4-c]pyridinyl; 3,4,4a,5,7,7a-Hexahydro-2H-pyrrolo[3,4-b][1,4]oxazinyl; 3-Oxo-1,5,6,7,8,8a-hexahydroimidazo[1,5-a]pyrazinyl; 3-Oxo-3a,4,5,6,7,7a-hexahydro-1H-pyrrolo[3,4-c]pyridinyl; Amino-(C₁₋₆-alkoxy)pyrrolidinyl, Amino-1,4-oxazepanyl; Aminohalogenpyrrolidinyl; Aminooxopyrrolidinyl oder Azetidinyl-C₁₋₆-alkoxy ist.

4. Verbindung nach Anspruch 1, wobei die Verbindung die Formel (Ia) aufweist 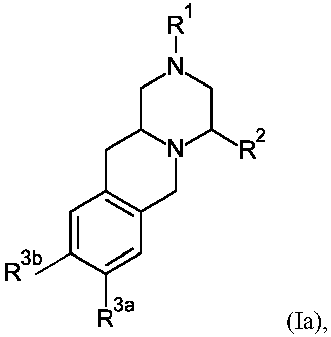 wobei
R¹ 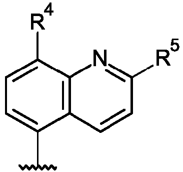 ist; wobei R⁴ Cyano ist, R⁵ H oder Deuterium ist;
R² C₁₋₆-Alkyl ist;
a) R^{3a} (C₁₋₆₋Alkyl)₂-amino-C₁₋₆-alkoxy, (Halogenpyrrolidinyl)amino; (Hydroxypyrrolidinyl)-C₁₋₆-alkylamino, (Morpholinyl)-C₁₋₆-alkylamino oder Amino-1,4-oxazepanyl ist;
R^{3b} H ist; oder
b) R^{3a} H ist;
R^{3b} (Amino-C₁₋₆-alkylcarbonyl)-2,5-diazabicyclo[2.2.1]heptanyl; (Amino-C₁₋₆-alkylcarbonyl)piperazinyl; (C₁₋₆-Alkoxypyrrolidinyl)amino, (C₁₋₆-Alkyl)₂-amino-C₁₋₆-alkoxy; (C₁₋₆-Alkylpyrrolidinylcarbonyl)piperazinyl, (Halogenpyrrolidinyl)amino; 1-Oxo-3a,4,5,6,7,7a-hexahydro-3H-pyrrolo[3,4-c]pyridinyl; 3,4,4a,5,7,7a-Hexahydro-2H-pyrrolo[3,4-b][1,4]oxazinyl; 3-Oxo-1,5,6,7,8,8a-hexahydroimidazo[1,5-a]pyrazinyl; 3-Oxo-3a,4,5,6,7,7a-hexahydro-1H-pyrrolo[3,4-c]pyridinyl; Amino-(C₁₋₆-alkoxy)pyrrolidinyl; Aminohalogenpyrrolidinyl; Aminooxopyrrolidinyl oder Azetidinyl-C₁₋₆-alkoxy ist;
oder ein pharmazeutisch unbedenkliches Salz davon.

5. Verbindung nach Anspruch 1, wobei die Verbindung die Formel (Ib) aufweist, 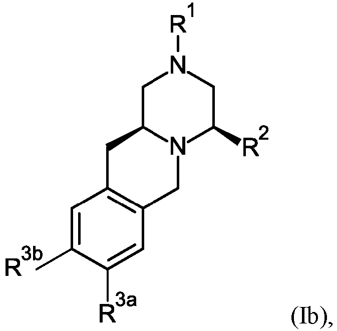 wobei
R¹ 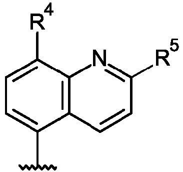 ist; wobei R⁴ Cyano ist, R⁵ H oder Deuterium ist;
R² C₁₋₆-Alkyl ist;
a) R^{3a} (C₁₋₆₋Alkyl)₂-amino-C₁₋₆-alkoxy, (Halogenpyrrolidinyl)amino; (Hydroxypyrrolidinyl)-C₁₋₆-alkylamino, (Morpholinyl)-C₁₋₆-alkylamino oder Amino-1,4-oxazepanyl ist;
R^{3b} H ist; oder
b) R^{3a} H ist;
R^{3b} (Amino-C₁₋₆-alkylcarbonyl)-2,5-diazabicyclo[2.2.1]heptanyl; (Amino-C₁₋₆-alkylcarbonyl)piperazinyl; (C₁₋₆-Alkoxypyrrolidinyl)amino, (C₁₋₆-Alkyl)₂-amino-C₁₋₆-alkoxy; (C₁₋₆-Alkylpyrrolidinylcarbonyl)piperazinyl, (Halogenpyrrolidinyl)amino; 1-Oxo-3a,4,5,6,7,7a-hexahydro-3H-pyrrolo[3,4-c]pyridinyl; 3,4,4a,5,7,7a-Hexahydro-2H-pyrrolo[3,4-b][1,4]oxazinyl; 3-Oxo-1,5,6,7,8,8a-hexahydroimidazo[1,5-a]pyrazinyl; 3-Oxo-3a,4,5,6,7,7a-hexahydro-1H-pyrrolo[3,4-c]pyridinyl; Amino-(C₁₋₆-alkoxy)pyrrolidinyl; Aminohalogenpyrrolidinyl; Aminooxopyrrolidinyl oder Azetidinyl-C₁₋₆-alkoxy ist;
oder ein pharmazeutisch unbedenkliches Salz davon.

6. Verbindung nach Anspruch 4 oder 5, wobei
R¹ 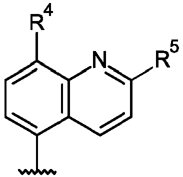 ist; wobei R⁴ Cyano ist, R⁵ H oder Deuterium ist;
R² C₁₋₆-Alkyl ist;
R^{3a} H ist;
R^{3b} (Amino-C₁₋₆-alkylcarbonyl)-2,5-diazabicyclo[2.2.1]heptanyl; (Amino-C₁₋₆-alkylcarbonyl)piperazinyl; (C₁₋₆-Alkoxypyrrolidinyl)amino, (C₁₋₆-Alkyl)₂-amino-C₁₋₆-alkoxy; (C₁₋₆-Alkylpyrrolidinylcarbonyl)piperazinyl, (Halogenpyrrolidinyl)amino; 1-Oxo-3a,4,5,6,7,7a-hexahydro-3H-pyrrolo[3,4-c]pyridinyl; 3,4,4a,5,7,7a-Hexahydro-2H-pyrrolo[3,4-b][1,4]oxazinyl; 3-Oxo-1,5,6,7,8,8a-hexahydroimidazo[1,5-a]pyrazinyl; 3-Oxo-3a,4,5,6,7,7a-hexahydro-1H-pyrrolo[3,4-c]pyridinyl; Amino-(C₁₋₆-alkoxy)pyrrolidinyl; Aminohalogenpyrrolidinyl; Aminooxopyrrolidinyl oder Azetidinyl-C₁₋₆-alkoxy ist;
oder ein pharmazeutisch unbedenkliches Salz davon.

7. Verbindung nach Anspruch 6, wobei R² Methyl ist.

8. Verbindung nach Anspruch 6, wobei R^{3b} (2-Amino-2-methylpropanoyl)-2,5-diazabicyclo[2.2.1]heptan-2-yl; (2-Amino-2-methylpropanoyl)piperazin-1-yl; (2-Methylpyrrolidin-2-carbonyl)piperazin-1-yl; (4-Fluorpyrrolidin-3-yl)amino; (4-Methoxypyrrolidin-3-yl)amino; (Azetidin-2-yl)methoxy; 1-Oxo-3a,4,5,6,7,7a-hexahydro-3H-pyrrolo[3,4-c]pyridin-2-yl; 2-(Dimethylamino)ethoxy; 3,4,4a,5,7,7a-Hexahydro-2H-pyrrolo[3,4-b][1,4]oxazin-6-yl; 3-Amino-4-fluorpyrrolidin-1-yl; 3-Amino-4-methoxypyrrolidin-1-yl; 3-Oxo-1,5,6,7,8,8a-hexahydroimidazo[1,5-a]pyrazin-2-yl; 3-Oxo-3a,4,5,6,7,7a-hexahydro-1H-pyrrolo[3,4-c]pyridin-2-yl oder 4-Amino-2-oxo-pyrrolidin-1-yl ist.

9. Verbindung nach Anspruch 6, wobei R^{3b} (C₁₋₆-Alkylpyrrolidinylcarbonyl)piperazinyl, 3,4,4a,5,7,7a-Hexahydro-2H-pyrrolo[3,4-b][1,4]oxazinyl; 3-Oxo-1,5,6,7,8,8a-hexahydroimidazo[1,5-a]pyrazinyl oder Azetidinyl-C₁₋₆-alkoxy ist.

10. Verbindung nach Anspruch 9, wobei R^{3b} (2-Methylpyrrolidin-2-carbonyl)piperazin-1-yl; 3,4,4a,5,7,7a-Hexahydro-2H-pyrrolo[3,4-b][1,4]oxazin-6-yl; 3-Oxo-1,5,6,7,8,8a-hexahydroimidazo[1,5-a]pyrazin-2-yl oder (Azetidin-2-yl)methoxy ist.

11. Verbindung nach Anspruch 4 oder 5, wobei
R¹ 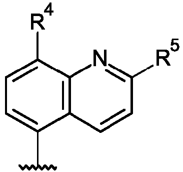 ist; wobei R⁴ Cyano ist, R⁵ H oder Deuterium ist;
R² C₁₋₆-Alkyl ist;
R^{3a} H ist;
R^{3b} (C₁₋₆-Alkylpyrrolidinylcarbonyl)piperazinyl, 3,4,4a,5,7,7a-Hexahydro-2H-pyrrolo[3,4-b][1,4]oxazinyl; 3-Oxo-1,5,6,7,8,8a-hexahydroimidazo[1,5-a]pyrazinyl oder Azetidinyl-C₁₋₆-alkoxy ist;
oder ein pharmazeutisch unbedenkliches Salz davon.

12. Verbindung nach Anspruch 11, wobei
R¹ 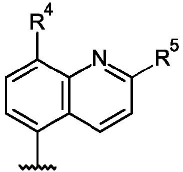 ist; wobei R⁴ Cyano ist, R⁵ H oder Deuterium ist;
R² Methyl ist;
R^{3a} H ist;
R^{3b} (2-Methylpyrrolidin-2-carbonyl)piperazin-1-yl; 3,4,4a,5,7,7a-Hexahydro-2H-pyrrolo[3,4-b][1,4]oxazin-6-yl; 3-Oxo-1,5,6,7,8,8a-hexahydroimidazo[1,5-a]pyrazin-2-yl oder (Azetidin-2-yl)methoxy ist;
oder ein pharmazeutisch unbedenkliches Salz davon.

13. Verbindung nach einem der Ansprüche 1 bis 12, wobei die Verbindung aus Folgenden ausgewählt ist:
5-[(4*R*,11a*S*)-8-(6-Amino-1,4-oxazepan-4-yl)-4-methyl-1,3,4,6,11,11a-hexahydropyrazino[1,2-b]isochinolin-2-yl]chinolin-8-carbonitril;
5-[(4*R*,11a*S*)-8-[2-[(3*S*)-3-Hydroxypyrrolidin-1-yl]ethylamino]-4-methyl-1,3,4,6,11,11a-hexahydropyrazino[1,2-b]isochinolin-2-yl]chinolin-8-carbonitril;
5-[(4*R*,11a*S*)-4-Methyl-8-[[(2*R*)-morpholin-2-yl]methylamino]-1,3,4,6,11,11a-hexahydropyrazino[1,2-b]isochinolin-2-yl]chinolin-8-carbonitril;
5-[(4*R*,11a*S*)-8-[[(3*S*,4*R*)-4-Fluorpyrrolidin-3-yl]amino]-4-methyl-1,3,4,6,11,11a-hexahydropyrazino[1,2-b]isochinolin-2-yl]chinolin-8-carbonitril;
5-[(4*R*,11a*S*)-8-[2-(Dimethylamino)ethoxy]-4-methyl-1,3,4,6,11,11a-hexahydropyrazino[1,2-b]isochinolin-2-yl]chinolin-8-carbonitril;
5-[(4*R*,11a*S*)-4-Methyl-9-[[(3*R*,4*S*)-4-fluoropyrrolidin-3-yl]amino]-1,3,4,6,11,11a-hexahydropyrazino[1,2-b]isochinolin-2-yl]chinolin-8-carbonitril;
5-[(4*R*,11a*S*)-9-[[(3*R*,4*S*)-4-Methoxypyrrolidin-3-yl]amino]-4-methyl-1,3,4,611,11a-hexahydropyrazino[1,2-b]isochinolin-2-yl]chinolin-8-carbonitril;
5-[(4*R*,11a*S*)-9-[(3*S*,4*R*)-3-Amino-4-fluorpyrrolidin-1-yl]-4-methyl-1,3,4,6,11,11a-hexahydropyrazino[1,2-b]isochinolin-2-yl]chinolin-8-carbonitril;
5-[(4*R*,11a*S*)-9-[(3*R*,4*S*)-3-Amino-4-methoxypyrrolidin-1-yl]-4-methyl-1,3,4,6,11,11a-hexahydropyrazino[1,2-b]isochinolin-2-yl]chinolin-8-carbonitril;
5-[(4*R*,11a*S*)-9-[(4a*S*,7a*S*)-3,4,4a,5,7,7a-Hexahydro-2*H*-pyrrolo[3,4-b][1,4]oxazin-6-yl]-4-methyl-1,3,4,6,11,11a-hexahydropyrazino[1,2-b]isochinolin-2-yl]chinolin-8-carbonitril;
5-[(4*R*,11a*S*)-9-[2-(Dimethylamino)ethoxy]-4-methyl-1,3,4,6,11,11a-hexahydropyrazino[1,2-b]isochinolin-2-yl]chinolin-8-carbonitril;
5-[(4*R*,11a*S*)-9-[[(2*S*)-Azetidin-2-yl]methoxy]-4-methyl-1,3,4,6,11,11a-hexahydropyrazino[1,2-b]isochinolin-2-yl]chinolin-8-carbonitril;
5-[(4*R*,11a*S*)-4-Methyl-9-[[(3*R*,4*S*)-4-fluorpyrrolidin-3-yl]amino]-1,3,4,6,11,11a-hexahydropyrazino[1,2-b]isochinolin-2-yl]chinolin-8-carbonitril;
5-[(4*R*,11a*S*)-4-Methyl-9-[4-[(2*S*)-2-methylpyrrolidin-2-carbonyl]piperazin-1-yl]-1,3,4,6,11,11a-hexahydropyrazino[1,2-b]isochinolin-2-yl]chinolin-8-carbonitril;
5-[(4*R*,11a*S*)-9-[(1*S*,4*S*)-5-(2-Amino-2-methylpropanoyl)-2,5-diazabicyclo[2.2.1]heptan-2-yl]-4-methyl-1,3,4,6,11,11a-hexahydropyrazino[1,2-b]isochinolin-2-yl]chinolin-8-carbonitril;
5-[(4*R*,11a*S*)-9-(4-Amino-2-oxopyrrolidin-1-yl)-4-methyl-1,3,4,6,11,11a-hexahydropyrazino[1,2-b]isochinolin-2-yl]chinolin-8-carbonitril;
5-[(4*R*,11a*S*)-4-Methyl-9-[(8a*S*)-3-oxo-1,5,6,7,8,8a-hexahydroimidazo[1,5-a]pyrazin-2-yl]-1,3,4,6,11,11a-hexahydropyrazino[1,2-b]isochinolin-2-yl]chinolin-8-carbonitril;
5-[(4*R*,11a*S*)-4-Methyl-9-[(8a*R*)-3-oxo-1,5,6,7,8,8a-hexahydroimidazo[1,5-a]pyrazin-2-yl]-1,3,4,6,11,11a-hexahydropyrazino[1,2-b]isochinolin-2-yl]chinolin-8-carbonitril;
5-[(4*R*,11a*S*)-4-Methyl-9-(3-oxo-3a,4,5,6,7,7a-hexahydro-1*H*-pyrrolo[3,4-c]pyridin-2-yl)-1,3,4,6,11,11a-hexahydropyrazino[1,2-b]isochinolin-2-yl]chinolin-8-carbonitril und
5-[(4*R*,11a*S*)-4-Methyl-9-(1-oxo-3a,4,5,6,7,7a-hexahydro-3*H-*pyrrolo[3,4-c]pyridin-2-yl)-1,3,4,6,11,11a-hexahydropyrazino[1,2-b]isochinolin-2-yl]chinolin-8-carbonitril;
oder ein pharmazeutisch unbedenkliches Salz, Enantiomer oder Diastereomer davon.

14. Verfahren zur Herstellung einer Verbindung nach einem der Ansprüche 1 bis 13, umfassend den folgenden Schritt:
a) die Reaktion einer Verbindung der Formel (V), 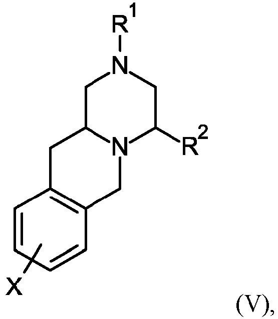 und R³-H über Buchwald-Hartwig-Aminierung oder Suzuki-Kupplung oder Stille-Kupplung oder Negishi-Kupplung; wobei R³-H ein Amin oder ein Alkohol ist;
wobei X Halogen oder eine Abgangsgruppe ist; R¹ bis R³ wie in einem der Ansprüche 1 bis 12 definiert sind.

15. Verbindung oder pharmazeutisch unbedenkliches Salz, Enantiomer oder Diastereomer nach einem der Ansprüche 1 bis 13 zur Verwendung als therapeutisch wirksame Substanz.

16. Pharmazeutische Zusammensetzung, umfassend eine Verbindung nach einem der Ansprüche 1 bis 13 und einen therapeutisch inerten Träger.

17. Verbindung oder pharmazeutisch unbedenkliches Salz, Enantiomer oder Diastereomer nach einem der Ansprüche 1 bis 13 zur Verwendung bei der Behandlung oder Prophylaxe von systemischem Lupus erythematodes oder Lupus-Nephritis.

## Revendications

1. Composé de formule (I), 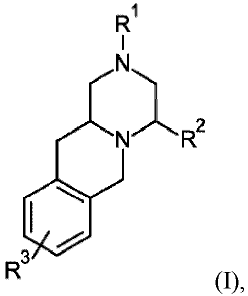 dans lequel 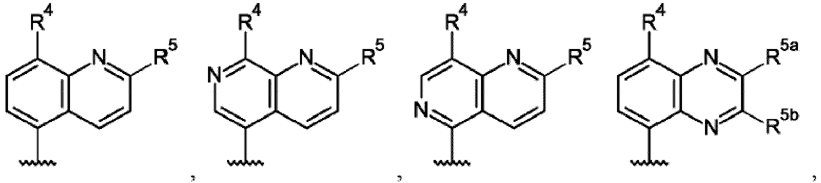
R¹ représente 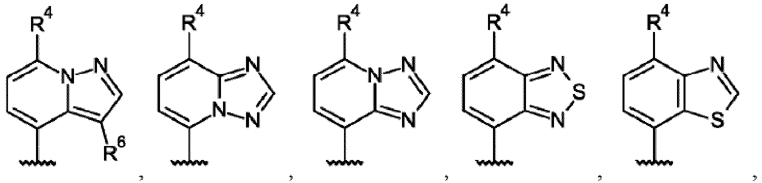 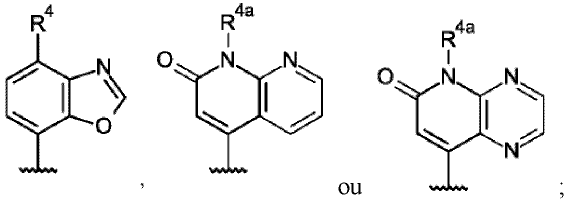 dans lequel R⁴ représente un alkyle en C₁₋₆, un alcoxy en C₁₋₆, un halogénoalkyle en C₁₋₆, un halogène, un nitro ou un cyano ; R^{4a} représente un alkyle en C₁₋₆ ou un cycloalkyle en C₃₋₇ ; R⁵*,* R^{5a} et R^{5b} sont indépendamment choisis parmi H et un deutérium ; R⁶ représente H ou un halogène ;
R² représente un alkyle en C₁₋₆ ;
R³ représente un hétérocyclyle non substitué ou substitué, un hétérocyclylamino, un hétérocyclylalkyle en C₁₋₆-amino ou un alcoxy en C₁₋₆ substitué ;
ou un sel pharmaceutiquement acceptable de celui-ci.

2. Composé selon la revendication 1, dans lequel R¹ représente 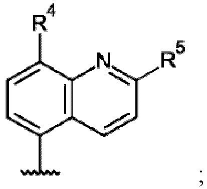 dans lequel R⁴ représente un cyano ; R⁵ représente H ou un deutérium.

3. Composé selon la revendication 2, dans lequel R³ représente un (aminoalkyle en C₁₋₆-carbonyl)-2,5-diazabicyclo[2.2.1]heptanyle ; un (aminoalkyle en C₁₋₆-carbonyl)pipérazinyle ; un (alcoxy en C₁₋₆-pyrrolidinyl)amino ; un (alkyle en C₁₋₆)₂aminoalcoxy en C₁₋₆ ; un (alkyle en C₁₋₆-pyrrolidinylcarbonyl)pipérazinyle ; un (halogénopyrrolidinyl)amino ; un (hydroxypyrrolidinyl)alkyle en C₁₋₆-amino ; un (morpholinyl)alkyle en C₁₋₆-amino ; un 1-oxo-3a,4,5,6,7,7a-hexahydro-3H-pyrrolo[3,4-c]pyridinyle ; un 3,4,4a,5,7,7a-hexahydro-2H-pyrrolo[3,4-b][1,4]oxazinyle ; un 3-oxo-1,5,6,7,8,8a-hexahydroimidazo[1,5-a]pyrazinyle ; un 3-oxo-3a,4,5,6,7,7a-hexahydro-1H-pyrrolo[3,4-c]pyridinyle ; un amino(alcoxy en C₁₋₆) pyrrolidinyle ; un amino-1,4-oxazépanyle ; un aminohalogénopyrrolidinyle ; un aminooxopyrrolidinyle ou un azétidinylalcoxy en C₁₋₆.

4. Composé selon la revendication 1, dans lequel le composé est de formule (Ia), 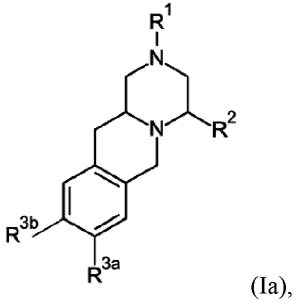 dans lequel
R¹ représente 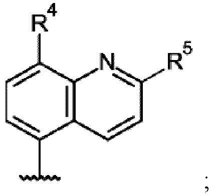 dans lequel R⁴ représente un cyano ; R⁵ représente H ou un deutérium ;
R² représente un alkyle en C₁₋₆ ;
a) R^{3a} représente un (alkyle en C₁₋₆)₂aminoalcoxy en C₁₋₆ ; un
(halogénopyrrolidinyl)amino ; un (hydroxypyrrolidinyl)alkyle en C₁₋₆-amino ; un (morpholinyl)alkyle en C₁₋₆-amino ou un amino-1,4-oxazépanyle ;
R^{3b} représente H ; ou
b) R^{3a} représente H ;
R^{3b} représente un (aminoalkyle en C₁₋₆-carbonyl)-2,5-diazabicyclo[2.2.1]heptanyle ; un (aminoalkyle en C₁₋₆-carbonyl)pipérazinyle ; un (alcoxy en C₁₋₆-pyrrolidinyl)amino ; un (alkyle en C₁₋₆)₂aminoalcoxy en C₁₋₆ ; un (alkyle en C₁₋₆-pyrrolidinylcarbonyl)pipérazinyle ; un (halogénopyrrolidinyl)amino ; un 1-oxo-3a,4,5,6,7,7a-hexahydro-3H-pyrrolo[3,4-c]pyridinyle ; un 3,4,4a,5,7,7a-hexahydro-2H-pyrrolo[3,4-b][1,4]oxazinyle ; un 3-oxo-1,5,6,7,8,8a-hexahydroimidazo[1,5-a] pyrazinyle ; un 3-oxo-3a,4,5,6,7,7a-hexahydro-1H-pyrrolo[3,4-c]pyridinyle ; un amino(alcoxy en C₁₋₆)pyrrolidinyle ; un aminohalogénopyrrolidinyle ; un aminooxopyrrolidinyle ou un azétidinylalcoxy en C₁₋₆ ;
ou un sel pharmaceutiquement acceptable de celui-ci.

5. Composé selon la revendication 1, dans lequel le composé est de formule (Ib), 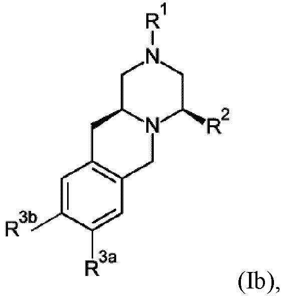 dans lequel
R¹ représente 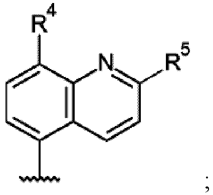 dans lequel R⁴ représente un cyano ; R⁵ représente H ou un deutérium ;
R² représente un alkyle en C₁₋₆ ;
a) R^{3a} représente un (alkyle en C₁₋₆)₂aminoalcoxy en C₁₋₆ ; un
(halogénopyrrolidinyl)amino ; un (hydroxypyrrolidinyl)alkyle en C₁₋₆-amino ; un (morpholinyl)alkyle en C₁₋₆-amino ou un amino-1,4-oxazépanyle ;
R^{3b} représente H ; ou
b) R^{3a} représente H ;
R^{3b} représente un (aminoalkyle en C₁₋₆-carbonyl)-2,5-diazabicyclo[2.2.1]heptanyle ; un (aminoalkyle en C₁₋₆-carbonyl)pipérazinyle ; un (alcoxy en C₁₋₆-pyrrolidinyl)amino ; un (alkyle en C₁₋₆)₂aminoalcoxy en C₁₋₆ ; un (alkyle en C₁₋₆-pyrrolidinylcarbonyl)pipérazinyle ; un (halogénopyrrolidinyl)amino ; un 1-oxo-3a,4,5,6,7,7a-hexahydro-3H-pyrrolo[3,4-c]pyridinyle ; un 3,4,4a,5,7,7a-hexahydro-2H-pyrrolo[3,4-b][1,4]oxazinyle ; un 3-oxo-1,5,6,7,8,8a-hexahydroimidazo[1,5-a] pyrazinyle ; un 3-oxo-3a,4,5,6,7,7a-hexahydro-1H-pyrrolo[3,4-c]pyridinyle ; un amino(alcoxy en C₁₋₆)pyrrolidinyle ; un aminohalogénopyrrolidinyle ; un aminooxopyrrolidinyle ou un azétidinylalcoxy en C₁₋₆ ;
ou un sel pharmaceutiquement acceptable de celui-ci.

6. Composé selon la revendication 4 ou 5, dans lequel
R¹ représente 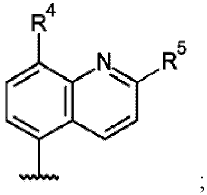 dans lequel R⁴ représente un cyano ; R⁵ représente H ou un deutérium ;
R² représente un alkyle en C₁₋₆ ;
R^{3a} représente H ;
R^{3b} représente un (aminoalkyle en C₁₋₆-carbonyl)-2,5-diazabicyclo[2.2.1]heptanyle ; un (aminoalkyle en C₁₋₆-carbonyl)pipérazinyle ; un (alcoxy en C₁₋₆-pyrrolidinyl)amino ; un (alkyle en C₁₋₆)₂aminoalcoxy en C₁₋₆ ; un (alkyle en C₁₋₆-pyrrolidinylcarbonyl)pipérazinyle ; un (halogénopyrrolidinyl)amino ; un 1-oxo-3a,4,5,6,7,7a-hexahydro-3H-pyrrolo[3,4-c]pyridinyle ; un 3,4,4a,5,7,7a-hexahydro-2H-pyrrolo[3,4-b][1,4]oxazinyle ; un 3-oxo-1,5,6,7,8,8a-hexahydroimidazo[1,5-a]pyrazinyle ; un 3-oxo-3a,4,5,6,7,7a-hexahydro-1H-pyrrolo[3,4-c]pyridinyle ; un amino(alcoxy en C₁₋₆)pyrrolidinyle ; un aminohalogénopyrrolidinyle ; un aminooxopyrrolidinyle ou un azétidinylalcoxy en C₁₋₆ ;
ou un sel pharmaceutiquement acceptable de celui-ci.

7. Composé selon la revendication 6, dans lequel R² représente un méthyle.

8. Composé selon la revendication 6, dans lequel R^{3b} représente un (2-amino-2-méthyl-propanoyl)-2,5-diazabicyclo[2.2.1]heptan-2-yle ; un (2-amino-2-méthyl-propanoyl)pipérazin-1-yle ; un (2-méthylpyrrolidine-2-carbonyl)pipérazin-1-yle ; un (4-fluoropyrrolidin-3-yl)amino ; un (4-méthoxypyrrolidin-3-yl)amino ; un (azétidin-2-yl)méthoxy ; un 1-oxo-3a,4,5,6,7,7a-hexahydro-3H-pyrrolo[3,4-c]pyridin-2-yle ; un 2-(diméthylamino)éthoxy ; un 3,4,4a,5,7,7a-hexahydro-2H-pyrrolo[3,4-b][1,4]oxazin-6-yle ; un 3-amino-4-fluoro-pyrrolidin-1-yle ; un 3-amino-4-méthoxy-pyrrolidin-1-yle ; un 3-oxo-1,5,6,7,8,8a-hexahydroimidazo[1,5-a]pyrazin-2-yle ; un 3-oxo-3a,4,5,6,7,7a-hexahydro-1H-pyrrolo[3,4-c]pyridin-2-yle ou un 4-amino-2-oxo-pyrrolidin-1-yle.

9. Composé selon la revendication 6, dans lequel R^{3b} représente un (alkyle en C₁₋₆-pyrrolidinylcarbonyl)pipérazinyle ; un 3,4,4a,5,7,7a-hexahydro-2H-pyrrolo[3,4-b][1,4]oxazinyle ; un 3-oxo-1,5,6,7,8,8a-hexahydroimidazo[1,5-a]pyrazinyle ou un azétidinylalcoxy en C₁₋₆.

10. Composé selon la revendication 9, dans lequel R^{3b} représente un (2-méthylpyrrolidine-2-carbonyl)pipérazin-1-yle ; un 3,4,4a,5,7,7a-hexahydro-2H-pyrrolo[3,4-b][1,4]oxazin-6-yle ; un 3-oxo-1,5,6,7,8,8a-hexahydroimidazo[1,5-a]pyrazin-2-yle ou un (azétidin-2-yl)méthoxy.

11. Composé selon la revendication 4 ou 5, dans lequel
R¹ représente 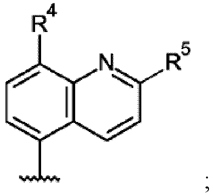 dans lequel R⁴ représente un cyano ; R⁵ représente H ou un deutérium ;
R² représente un alkyle en C₁₋₆ ;
R^{3a} représente H ;
R^{3b} représente un (alkyle en C₁₋₆-pyrrolidinylcarbonyl)pipérazinyle ; un 3,4,4a,5,7,7a-hexahydro-2H-pyrrolo[3,4-b][1,4]oxazinyle ; un 3-oxo-1,5,6,7,8,8a-hexahydroimidazo[1,5-a]pyrazinyle ou un azétidinylalcoxy en C₁₋₆ ;
ou un sel pharmaceutiquement acceptable de celui-ci.

12. Composé selon la revendication 11, dans lequel
R¹ représente 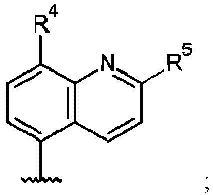 dans lequel R⁴ représente un cyano ; R⁵ représente H ou un deutérium ;
R² représente un méthyle ;
R^{3a} représente H ;
R^{3b} représente un (2-méthylpyrrolidine-2-carbonyl)pipérazin-1-yle ; un 3,4,4a,5,7,7a-hexahydro-2H-pyrrolo[3,4-b][1,4]oxazin-6-yle ; un 3-oxo-1,5,6,7,8,8a-hexahydroimidazo[1,5-a]pyrazin-2-yle ou un (azétidin-2-yl)méthoxy ;
ou un sel pharmaceutiquement acceptable de celui-ci.

13. Composé selon l'une quelconque des revendications 1 à 12, dans lequel le composé est choisi parmi :
le 5-[(4*R*,11a*S*)-8-(6-amino-1,4-oxazépan-4-yl)-4-méthyl-1,3,4,6,11,11a-hexahydropyrazino[1,2-b]isoquinoléin-2-yl]quinoléine-8-carbonitrile ;
le 5-[(4*R*,11a*S*)-8-[2-[(3*S*)-3-hydroxypyrrolidin-1-yl]éthylamino]-4-méthyl-1,3,4,6,11,11a-hexahydropyrazino[1,2-b]isoquinoléin-2-yl]quinoléine-8-carbonitrile ;
le 5-[(4*R*,11a*S*)-4-méthyl-8-[[(2*R*)-morpholin-2-yl]méthylamino]-1,3,4,6,11,11a-hexahydropyrazino[1,2-b]isoquinoléin-2-yl]quinoléine-8-carbonitrile ;
le 5-[(4*R*,11a*S*)-8-[[(3*S*,4*R*)-4-fluoropyrrolidin-3-yl]amino]-4-méthyl-1,3,4,6,11,11a-hexahydropyrazino[1,2-b]isoquinoléin-2-yl]quinoléine-8-carbonitrile ;
le 5-[(4*R*,11a*S*)-8-[2-(diméthylamino)éthoxy]-4-méthyl-1,3,4,6,11,11a-hexahydropyrazino[1,2-b]isoquinoléin-2-yl]quinoléine-8-carbonitrile ;
le 5-[(4*R*,11a*S*)-4-méthyl-9-[[(3*R*,4*S*)-4-fluoropyrrolidin-3-yl]amino]-1,3,4,6,11,11a-hexahydropyrazino[1,2-b]isoquinoléin-2-yl]quinoléine-8-carbonitrile ;
le 5-[(4*R*,11a*S*)-9-[[(3*R*,4*S*)-4-méthoxypyrrolidin-3-yl]amino]-4-méthyl-1,3,4,6,1l,11a-hexahydropyrazino[1,2-b]isoquinoléin-2-yl]quinoléine-8-carbonitrile ;
le 5-[(4*R*,11a*S*)-9-[(3*S*,4*R*)-3-amino-4-fluoro-pyrrolidin-1-yl]-4-méthyl-1,3,4,6,11,11a-hexahydropyrazino[1,2-b]isoquinoléin-2-yl]quinoléine-8-carbonitrile ;
le 5-[(4*R*,11a*S*)-9-[(3*R*,4*S*)-3-amino-4-méthoxy-pyrrolidin-1-yl]-4-méthyl-1,3,4,6,11,11a-hexahydropyrazino[1,2-b]isoquinoléin-2-yl]quinoléine-8-carbonitrile ;
le 5-[(4*R*,11a*S*)-9-[(4a*S*,7a*S*)-3,4,4a,5,7,7a-hexahydro-2*H*-pyrrolo[3,4-b][1,4]oxazin-6-yl]-4-méthyl-1,3,4,6,11,11a-hexahydropyrazino[1,2-b]isoquinoléin-2-yl]quinoléine-8-carbonitrile ;
le 5-[(4*R*,11a*S*)-9-[2-(diméthylamino)éthoxy]-4-méthyl-1,3,4,6,11,11a-hexahydropyrazino[1,2-b]isoquinoléin-2-yl]quinoléine-8-carbonitrile ;
le 5-[(4*R*,11a*S*)-9-[[(2*S*)-azétidin-2-yl]méthoxy]-4-méthyl-1,3,4,6,11,11a-hexahydropyrazino[1,2-b]isoquinoléin-2-yl]quinoléine-8-carbonitrile ;
le 5-[(4*R*,11a*S*)-4-méthyl-9-[[(3*R*,4*S*)-4-fluoropyrrolidin-3-yl]amino]-1,3,4,6,11,11a-hexahydropyrazino[1,2-b]isoquinoléin-2-yl]quinoléine-8-carbonitrile ;
le 5-[(4*R*,11a*S*)-4-méthyl-9-[4-[(2*S*)-2-méthylpyrrolidine-2-carbonyl]pipérazin-1-yl]-1,3,4,6,11,11a-hexahydropyrazino[1,2-b]isoquinoléin-2-yl]quinoléine-8-carbonitrile ;
le 5-[(4*R*,11a*S*)-9-[(1*S*,4*S*)-5-(2-amino-2-méthyl-propanoyl)-2,5-diazabicyclo[2.2.1]heptan-2-yl]-4-méthyl-1,3,4,6,11,11a-hexahydropyrazino[1,2-b]isoquinoléin-2-yl]quinoléine-8-carbonitrile ;
le 5-[(4*R*,11a*S*)-9-(4-amino-2-oxo-pyrrolidin-1-yl)-4-méthyl-1,3,4,6,11,11a-hexahydropyrazino[1,2-b]isoquinoléin-2-yl]quinoléine-8-carbonitrile ;
le 5-[(4*R*,11a*S*)-4-méthyl-9-[(8a*S*)-3-oxo-1,5,6,7,8,8a-hexahydroimidazo[1,5-a]pyrazin-2-yl]-1,3,4,6,11,11a-hexahydropyrazino[1,2-b]isoquinoléin-2-yl]quinoléine-8-carbonitrile;
le 5-[(4*R*,11a*S*)-4-méthyl-9-[(8a*R*)-3-oxo-1,5,6,7,8,8a-hexahydroimidazo[1,5-a]pyrazin-2-yl]-1,3,4,6,11,11a-hexahydropyrazino[1,2-b]isoquinoléin-2-yl]quinoléine-8-carbonitrile;
le 5-[(4*R*,11a*S*)-4-méthyl-9-(3-oxo-3a,4,5,6,7,7a-hexahydro-1*H*-pyrrolo[3,4-c]pyridin-2-yl)-1,3,4,6,11,11a-hexahydropyrazino[1,2-b]isoquinoléin-2-yl]quinoléine-8-carbonitrile ; et
le 5-[(4*R*,11a*S*)-4-méthyl-9-(1-oxo-3a,4,5,6,7,7a-hexahydro-3*H*-pyrrolo[3,4-c]pyridin-2-yl)-1,3,4,6,11,11a-hexahydropyrazino[1,2-b]isoquinoléin-2-yl]quinoléine-8-carbonitrile ;
ou un sel pharmaceutiquement acceptable, un énantiomère ou un diastéréoisomère de ceux-ci.

14. Procédé de préparation d'un composé selon l'une quelconque des revendications 1 à 13 comprenant l'étape suivante :
a) la réaction du composé de formule (V), 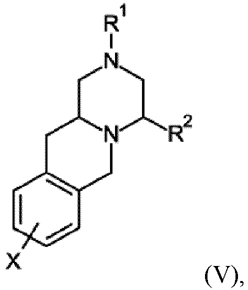 et de R³-H par amination de Buchwald-Hartwig ou couplage de Suzuki ou couplage de Stille ou couplage de Negishi ; dans lequel R³-H représente une amine ou un alcool ;
dans lequel X représente un halogène ou un groupe partant ; R¹ à R³ sont tels que définis dans l'une quelconque des revendications 1 à 12.

15. Composé ou sel pharmaceutiquement acceptable, énantiomère ou diastéréoisomère selon l'une quelconque des revendications 1 à 13 pour une utilisation comme substance thérapeutiquement active.

16. Composition pharmaceutique comprenant un composé selon l'une quelconque des revendications 1 à 13 et un véhicule thérapeutiquement inerte.

17. Composé ou sel pharmaceutiquement acceptable, énantiomère ou diastéréoisomère selon l'une quelconque des revendications 1 à 13 pour une utilisation dans le traitement ou la prophylaxie du lupus érythémateux disséminé ou de la néphropathie lupique.
